# EUROPEAN PATENT APPLICATION

(11) **EP 1 380 296 A1**
(43) Date of publication of application: **14.01.2004**
(21) Application number: 02708674.3
(22) Date of filing: 27.03.2002
(51) Int. Cl.: A61K 31/437

(54) **HSP INDUCTOR**

(30) Priority: 28.03.2001 JP 2001092704
(71) Applicant: Takeda Chemical Industries, Ltd., Osaka-shi, Osaka 541-0045 (JP)
(72) Inventor: TERASHITA, Zen-ichi, Toyonaka-shi, Osaka 560-0085 (JP); NARUO, Ken-ichi, Sanda-shi, Hyogo 669-1535 (JP); UCHIKAWA, Osamu, Kobe-shi, Hyogo 655-0002 (JP); NAKANISHI, Atsushi, Tsukuba-shi, Ibaraki 305-0025 (JP)
(74) Representative: von Kreisler, Alek, Dipl.-Chem.
(86) International application number: PCT/JP2002/002946
(87) International publication number: WO 2002/078705

(57) **Abstract**

An agent for inducing HSP, comprising a compound represented by the formula (I): 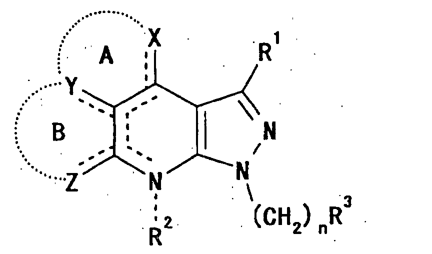 wherein R¹ is a hydrogen atom, a hydrocarbon group which may be substituted, etc.; R² is absent, a hydrogen atom or a hydrocarbon group which maybe substituted; R³ is a heterocyclic group which may be substituted; X, Y and Z are, respectively, a hydrogen, a halogen, a nitrile, a hydrocarbon group which may be substituted, or X and Y may bind to each other to form ring A, or Y and Z may bind to each other to form ring B; bond portions indicated by a solid line and a broken line are, respectively, a single bond or a double bond, and bond portions indicated by a broken line are, respectively, a single bond or absent; ring A is a homocyclic or heterocyclic 5- to 7-membered ring which may be substituted; ring B is a homocyclic or heterocyclic 5- to 7-membered ring which may be substituted; and n is an integer of 0 or 1,
or a salt thereof;

## Description

### Technical Field

The present invention relates to an agent for inducing HSP; a pharmaceutical composition which comprises a combination of a compound having HSP inducing activity and a compound having an activity capable of causing a tissue disorder; a pharmaceutical composition which comprises a combination of a compound having HSP inducing activity and a compound having COX inhibiting activity; a pharmaceutical composition which decreases a tissue disorder, comprising a compound having HSP inducing activity and an activity capable of causing a tissue disorder; a pharmaceutical composition which decreases a tissue disorder caused by a compound having COX inhibiting activity, which is useful for the prophylaxis and/or treatment of inflammatory disease, arthritis, rheumatism, rheumatoid arthritis, osteoarthritis or the like; and the like.

### Background Art

Various studies relating to Non-Steroidal AntiInflammatory Drugs (NSAIDs) in recent years have progressively clarified the relationship between the action point and action mechanism thereof, and side effects (e.g., tissue disorder in gastrointestinal tract, kidney and the like) thereof. For example, Vane and his collaborators reported the details of pharmacological action of aspirin in 1971 (Nature (New Biol.), 231, 232 (1971)), which made it clear that antipyretic action, analgesic action and anti-inflammatory action of aspirin and other non-steroidal anti-inflammatory drugs are the effects of inhibition of prostaglandin production.

Prostaglandin (PG) was discovered in 1930s, and its chemical structure was reported in 1960s. It was in 1972 that a report documented that prostaglandin is produced from arachidonic acid by incubation with an enzyme (oxygenase such as cyclooxygenase) contained in a homogenate of seminal vesicle from sheep (Adv. Prostagl. and Thromb. Res., 4; pp. 1-25 (1978)). Thereafter, a lot of work has been done to clarify the properties of cyclooxygenase (COX) as an enzyme, and its isozyme, COX-2, was found in 1991 (J. Biol. Chem., 266, 12866-12872 (1991)).

While the action of what is called non-steroidal anti-inflammatory drugs has now been mostly explained with the inhibition of prostaglandin production, these drugs problematically show strong side effects such as peptic ulcer in the gastrointestinal tract and kidney disorder. Currently, it is considered that these side effects are caused by the inhibition of COX-1 that homeostatically acts on the physiological functions, but the mechanism has not been clarified as yet.

Based on the foregoing understanding, celecoxib, rofecoxib and the like, having high selectivity to COX-2, have been developed and marketed as medicaments associated with fewer gastrointestinal disorders.

However, COX-2 as well as COX-1 are constitutively expressed in the kidneys, wherein, generally, COX-1 is considered to mainly act for the production of prostaglandins necessary for maintaining physiological functions, and COX-2 is considered to act for the biophylaxis such as inflammation and cell growth.

Furthermore, in view of experiments using COX-2 knockout mouse, a risk of causing kidney disorders by the administration of a COX-2 inhibitor has been pointed out, and it has been moreover reported that gastrointestinal disorders are aggravated by the use of a COX-2 inhibitor (Cell, 83, 345-348 (1995)). On the other hand, it has been found that prostaglandin produced by COX-2 acts for tissue repair and neovascularization (Trend Pharmacol. Sci., 20, 4-6 (1999)). Ir addition, COX-2 is believed to play an important role in the regulation of sodium and secretion of renin, since COX-2 is expressed in interstitial cells of the macula densa and the renal medulla, and the expression is induced by sodium restriction or decreased blood flow in the kidney (J. Clin. Invest., 94, 2504-2510 (1994)).

From the above aspects, it is also believed that COX-2 selective inhibitors should be administered carefully.

As COX inhibitors, for example,
(1) Classical NSAIDs
   Diclofenac, Indomethacin, Aspirin, Ibuprofen, Ketoprophen, Piroxicam, etc.;
(2) COX-2 selective inhibitors
   Celecoxib, Rofecoxib, MK-663, Valdecoxib, SC-57666, JTE-522, S-2474, SC-57666, etc.;
(3) dual inhibitors
   ML-3000, p54 (COX inhibitor & 5-lipoxygenase inhibitor), etc.;
(4) NO (Nitric oxide)-releasing NSAIDs;
   and the like have been heretofore reported.

Incidentally, Heat Shock Protein (HSP) is a protein induced by cells on exposure to stresses such as heat stimulation and the like, which is considered to be a substance necessary for survival of the cell under high temprature. HSP is also induced by various non-specific stresses (alcohol, oxidative stress etc.) other than heat stimulation, and it is believed that this HSP induction confers on cells the resistance non-specific to various stresses (Inflammatory, 20, NO. 3, 189-201 (2000)).

It has been also reported that, in gastric mucosa cell exposured to various stresses, any stress (alcohol, acid, active oxygen, anti-inflammatory drug etc.) on gastric mucosa induces apoptosis, and the apoptosis is inhibited by HSP induction. (Inflammatory, 20, NO. 3, 189-201 (2000)). These results suggest that an HSP inducer is an ideal agent for protecting gastric mucosa from any stress.

Furthermore, it have been reported that HSP27, one of HSPs, inhibits cell death (Nature Cell Biology, 2, .645-652 (2000), Molecular and Cellular Biology, 20, 7602-7612 (2000)).

As mentioned above, HSP is one of the stress proteins, which is induced by cells for the survival thereof under a stress environment, and various molecules have been heretofore known as HSPs, such as HSP100, HSP90, HSP70, HSP60, HSP56, HSP47 and HSP27 (Handbook of Experimental Pharmacology, 136, pp. 1-7 (1998)).

As such agent for inducing HSP, geranylgeranylacetone (GGA) (trade name: Selbex) is known, which is a drug for protecting gastric mucosa. This GGA reportedly induces HSP on gastric mucosa cells *in vitro* and *in vivo* by activation of transcriptional factor for HSP gene (Gastroenterology, 111, 345.-357 (1996)). In addition, it has been reported that cytotoxicity caused by indomethacin is decreased by treating gastric mucosa cell with geranylgeranylacetone previously (Digestive Diseases & Science, 45, 1674-1679 (2000)).

### Disclosure of the Invention

The present invention aims to develop agents such as those that do not express tissue disorders in the gastrointestinal tract, the kidneys and the like of patients, even when a compound that can induce tissue disorder, such as a compound having COX inhibitory activity effective for prophylaxis or treatment, is administered for the prophylaxis or treatment of inflammatory disease, arthritis, rheumatism, rheumatoid arthritis, osteoarthritis, other diseases and the like.

The present inventors have found for the first time that tissue disorders in the gastrointestinal tract, kidneys and the like of patients suffering from inflammatory disease, arthritis, rheumatism, rheumatoid arthritis or osteoarthritis can be reduced by allowing, during administration of a COX inhibitor, an HSP inducer to act to the extent that the tissue disorders cannot be expressed. The present inventors have further proceeded with the study based on these findings, and completed the present invention.

Accordingly, the present invention relates to:
(1) an agent for inducing HSP, comprising a compound represented by the formula (I): 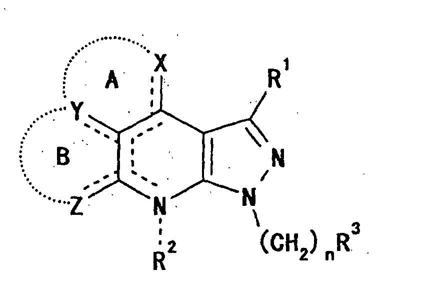 wherein
   - R¹: is a hydrogen atom, a hydrocarbon group which may be substituted, an amino group which may be substituted, a sulfur atom which may be substituted or a carboxyl group which may be esterified or amidated;
   - R²: is absent, a hydrogen atom or a hydrocarbon group which may be substituted;
   - R³: is a heterocyclic group which may be substituted;
   - X, Y and Z: are, respectively, a hydrogen, a halogen, a nitrile, a hydrocarbon group which may be substituted, a carboxyl group which may be esterified or amidated, an acyl group which may be substituted, -NR⁴R⁵, an oxygen atom, -OR⁴ a sulfur atom or -SR⁴ (R⁴ and R⁵ are, respectively, a hydrogen atom, a hydrocarbon group which may be substituted, or a heterocyclic group which may be substituted, or may bind to each other to form a cyclic amino group together with the nitrogen atom they bind with), or X and Y may bind to each other to form ring A, or Y and Z may bind to each other to form ring B;
   bond portions indicated by a solid line and a broken line are, respectively, a single bond or a double bond, and bond portions indicated by a broken line are, respectively, a single bond or absent;
   - ring A: is a homocyclic or heterocyclic 5- to 7-membered ring which may be substituted;
   - ring B: is a homocyclic or heterocyclic 5- to 7-membered ring which may be substituted; and
   - n: is an integer of 0 or 1,
   or a salt thereof;
(2) an agent for inducing HSP, comprising 3-methyl-1-(2-pyridinyl)-6-trifluoromethyl-1,9-dihydro-4H-pyrazolo[3,4-b]quinolin-4-one or a salt thereof;
(3) an agent for inducing HSP, comprising 6,7-difluoro-3-methyl-1-(2-pyridinyl)-1,9-dihydro-4H-pyrazolo[3,4-b]quinolin-4-one or a salt thereof;
(4) an agent for inducing HSP, comprising 6-chloro-7-fluoro-3-methyl-1-(2-pyridinyl)-1,9-dihydro-4H-pyrazolo[3,4-b]quinolin-4-one or a salt thereof;
(5) an agent for inducing HSP, comprising 6-chloro-1-(2-pyridinyl)-1,9-dihydro-4H-pyrazolo[3,4-b]quinolin-4-one or a salt thereof;
(6) the agent of any of (1) to (5) described above, which is an agent for ameliorating, or an agent for decreasing the progression of, a tissue disorder;
(7) the agent of (6) described above, wherein the tissue disorder is a digestive tract disorder or a kidney disorder;
(8) a method for inducing HSP, which comprises administering an effective amount of the compound of any of (1) to (5) described above or a salt thereof to a mammal;
(9) use of the compound of any of (1) to (5) described above or salt thereof, for the production of an agent for inducing HSP;
(10) a pharmaceutical composition which decreases a tissue disorder, comprising a compound having HSP inducing activity and an activity capable of causing the tissue disorder;
(11) the composition of (10) described above, wherein the activity capable of causing the tissue disorder is COX inhibiting activity;
(12) the composition of (11) described above, which is used for inhibiting COX;
(13) the composition of any of (10) to (12) described above, which is an agent for ameliorating, or an agent for decreasing the progression of, the tissue disorder;
(14) the composition of (13) described above, wherein the tissue disorder is a gastrointestinal tract disorder or a kidney disorder;
(15) an agent for ameliorating, or an agent for decreasing the progression of, a gastrointestinal tract disorder or a kidney disorder, which comprises a compound having HSP inducing activity and an activity capable of causing the gastrointestinal tract disorder or the kidney disorder;
(16) the composition of (11) or (12) described above, which is an agent for the prophylaxis or treatment of inflammatory disease, arthritis, rheumatism, rheumatoid arthritis or osteoarthritis;
(17) an agent for the prophylaxis or treatment of inflammatory disease, arthritis, rheumatism, rheumatoid arthritis or osteoarthritis, which decreases a gastrointestinal tract or kidney disorder, said agent comprising a compound having HSP inducing activity and COX inhibiting activity;
(18) the composition of (101 described above which decreases a tissue disorder caused by an activity capable of causing the tissue disorder, by about 60% or more;
(19) the composition of (11) described above, which decreases a tissue disorder caused by COX inhibiting activity, by about 60% or more;
(20) the composition of (10) described above, wherein the compound is represented by the formula (I): 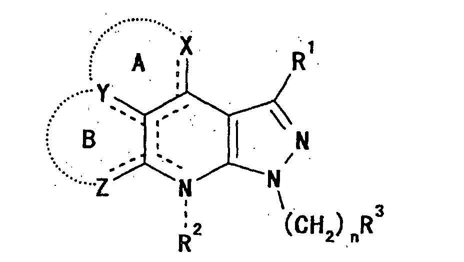 wherein
   - R¹: is a hydrogen atom, a hydrocarbon group which may be substituted, an amino group which may be substituted, a sulfur atom which may be substituted or a carboxyl group which may be esterified or amidated;
   - R²: is absent, a hydrogen atom or a hydrocarbon group which may be substituted
   - R³: is a heterocyclic group which may be substituted;
   - X, Y and Z: are, respectively, a hydrogen, a halogen, a nitrile, a hydrocarbon group which may be substituted, a carboxyl group which may be esterified or amidated, an acyl group which may be substituted, -NR⁴R⁵, an oxygen atom, -OR⁴, a sulfur atom or -SR⁴ (R⁴ and R⁵ are, respectively, a hydrogen atom, a hydrocarbon group which may be substituted, or a heterocyclic group which may be substituted, or may bind to each other to form a cyclic amino group together with the nitrogen atom they bind with), or X and Y may bind to each other to form ring A, or Y and Z may bind to each other to form ring B;
   bond portions indicated by a solid line and a broken line are, respectively, a single bond or a double bond, and bond portions indicated by a broken line are, respectively, a single bond or absent;
   - ring A: is a homocyclic or heterocyclic 5- to 7-membered ring which may be substituted;
   - ring B: is a homocyclic or heterocyclic 5- to 7-membered ring which may be substituted; and
   - n: is an integer of 0 or 1,
   or a salt thereof;
(21) the composition of (20) described above, wherein the compound is 3-methyl-1-(2-pyridinyl)-6-trifluoromethyl-1,9-dihydro-4H-pyrazolo[3,4-b]quinolin-4-one or a salt thereof;
(22) the composition of (20) described above, wherein the compound is 6,7-difluoro-3-methyl-1-(2-pyridinyl)-1,9-dihydro-4H-pyrazolo[3,4-b]quinolin-4-one or a salt thereof;
(23) the composition of (20) described above, wherein the compound is 6-chloro-7-fluoro-3-methyl-1-(2-pyridinyl)-1,9-dihydro-4H-pyrazolo[3,4-b]quinolin-4-one or a salt thereof;
(24) the composition of (20) described above, wherein the compound is 6-chloro-1-(2-pyridinyl)-1,9-dihydro-4H-pyrazolo[3,4-b)quinolin-4-one or a salt thereof;
(25) a method for decreasing a tissue disorder, which comprises administering an effective amount of a compound having HSP inducing activity and an activity capable of causing the tissue disorder to a mammal;
(26) use of a compound having HSP inducing activity and an activity capable of causing a tissue disorder, for the production of a pharmaceutical composition which decreases the tissue disorder;
(27) a method for decreasing a tissue disorder caused by COX inhibiting activity, which comprises administering an effective amount of a compound having HSP inducing activity and COX inhibiting activity to a mammal;
(28) use of a compound having HSP inducing activity and COX inhibiting activity, for the production of a pharmaceutical composition which decreases an activity capable of causing a tissue disorder caused by COX inhibiting activity;
(29) a pharmaceutical composition comprising a combination of a compound having HSP inducing activity (preferably except GGA) and a compound having an activity capable of causing a tissue disorder (preferably except indomethacin);
(30) a pharmaceutical composition comprising a combination of a compound having HSP inducing activity and a compound having COX inhibiting activity;
(31) the composition of (29) or (30) described above, which is an agent for ameliorating, or an agent for decreasing the progression of, the tissue disorder;
(32) the composition of (31) described above, wherein the tissue disorder is a gastrointestinal tract or kidney disorder;
(33) a method for decreasing a tissue disorder, which comprises administering an effective amount of a compound having HSP inducing activity and an effective amount of a compound having an activity capable of causing the tissue disorder to a mammal;
(34) use of a compound having HSP inducing activity and a compound having an activity capable of causing a tissue disorder, for the production of a pharmaceutical composition which decreases the tissue disorder;
(35) a method for decreasing a tissue disorder caused by COX inhibiting activity, which comprises administering an effective amount of a compound having HSP inducing activity and an effective amount of a compound having COX inhibiting activity to a mammal;
(36) use of a compound having HSP inducing activity and a compound having COX inhibiting activity, for the production of a pharmaceptical composition which decreases an activity capable of causing a tissue disorder caused by COX inhibiting activity; and the like.

### Detailed Description of the Invention

The "Compound having HSP inducing activity" used in the present invention may be any compound as far as it is a compound having HSP inducing activity.

As used herein, the HSP inducing activity is meant presentation of clear HSP inducing activity (preferably, HSP27 inducing activity) at the dose of, for example, 100 mg/kg or less in *vivo.* Specifically, as described below in Test Example 4, HSP27 inducing activity at a dose of 100 mg/kg or less is preferable. That is, HSP27 inducing-activity of the level 1.5 times or higher relative to control-group is preferable Moreover, HSP27 inducing activity of the level 2.0 times or higher relative to control group is more preferable. It is desirable to show an activity to decrease tissue disorders such as digestive tract disorder and kidney disorder, which are caused by a compound having an activity capable of causing the tissue disorder (e.g., a compound having COX inhibiting activity, etc.), by about 60% or more as a result of the expression of the HSP inducing activity (preferably HSP27 inducing activity).

The "compound having an activity capable of causing a tissue disorder" used in the present invention may be any compound having an activity capable of causing a tissue disorder as a side effect. Examples of the "compound having an activity capable of causing a tissue disorder" include anti-inflammatory drugs (e.g., COX-1 inhibitor, COX-2 inhibitor etc.); steroidal anti-inflammatory drugs; antirheumatic drugs (e.g., enbrel, remikade, methotrexate, tacrolimus, ^{p}38 MAP. kinase, cell differentiation promotor etc.); antianxiety drugs; antidepressants;,drugs for treating schizophrenia; choline esterase inhibitors (e.g:, aricept etc.); drugs for treating Alzheimer's disease (e.g., β-amyloid protein producing or secreting inhibitor etc.); various cytokine preparations; drugs for treating Parkinson's disease; general or local anesthetics; various drops; anti-allergic drugs; various anti-asthmatic drugs (e.,g., thromboxane antagonist, thromboxane synthesis inhibitor, leucotriene antagonist, leucotriene synthesis inhibitor etc.); drugs for treating COPD (e.g., PDEIV inhibitor etc.); antitussives ; cardiac stimulants; antiarrhythmic drugs; antihypertensive drugs (e.g., ACE inhibitor, All antagonist, Ca antagonist, β-blocker, K channel opening drug, α-blocker etc.); endothelin antagonist; vasodilators, drugs for treating cerebrovascular disorder; drugs for treating acute cerebral infarction; antithrombotic drugs (e.g., thrombolytic drug, anti-platelet drug etc.); drugs for treating hyperlipidemia (e.g., statins (atrovastatin, etc.), fibrates (fenofibrate, etc.) etc.; various adhesive protein inhibitors; respiratory stimulants; antiulcer drugs (e.g., PPI inhibitors (lansoprazole, omeprazole, etc.), H2 antagonist, etc.); antiemetics; drugs for improving gastrointestinal tract function; drugs for treating irritable bowel syndrome; various hormone preparations; drugs for treating prostatomegaly; antimicrobial drug; artificial blood; anticoagulants (e.g., small heparin, arga-troban, hirudin, etc.); anti-platelet drugs (e.g., clopidogrel, ticlopidine, aspirin, cilostazole, ozagrel, GPIIb/IIIa antagonist, vWF antagonist etc.); thrombolytic drugs (e.g., tPA, tPA mutein, prourokinase, urokinase, etc.) ; hematopoietics (e.g., erythropoietin and mutein thereof etc.); substance for promoting organ regeneration (e.g., liver regeneration promotor (HGF, etc.) etc.); drugs for treating hepatic disease (e.g., interferon family, vaccine for hepatitis, etc.); drugs for treating gout (e.g., xanthine oxidase inhibitor, etc.); drugs for treating diabetes (e.g., nuclear receptor activator (actos etc.), drugs for inhibiting glucose absorption (voglibose etc.), insulin preparation, etc.); drugs for treating diabetic complications (e.g., Y-128, captopril, aldose reductase inhibitor, etc.); antiobestic drugs (e.g., sibtramine, etc.); drugs for treating pancreatitis; immunosuppressants (e.g., tacrolimus, etc.); drugs for treating osteoporosis (e.g., risedronate, PTH, calcitonin, estrogen preparation, etc.); anti-tumor drugs (e.g., 5FU, adriamycin, taxol, alkylating drug, platinum complex, metabolite antagonist, topoisomerases, microtubule protein agonist, anti-hormone, antibiotics, molecular-targeting anti-cancer drug (ZD1839), cytokine, monoclonal antibody (herceptin) etc.); radioisotope drugs; antibiotics; anti-AIDS drugs; vaccines; narcotics (e.g., morphine, etc.) and the like, and include compounds capable of causing a tissue disorders, such as gastrointestinal tract disorder, kidney disorder, liver disorder, pancreatic disorder, lung disorder, testis disorder, blood cells disorder, cardiac muscle disorder, striated muscle disorder, nerve disorder, retinal disorder, skin disorder, sensory organ disorder (disorder of eye, ear and the like), cerebrovascular disorder (preferably, disorder of gastrointestinal tract and kidney, etc.). Among these compounds, a compound having an activity to treat Alzheimer's disease (e.g., compound having an activity to produce and/or secrete β-amyloid protein, etc.); a compound having an activity to treat COPD (e.g., a compound having PDEIV inhibiting activity, etc.); a compound having a cardiac stimulation activity; a compound having anti-hypertensive activity (e.g., a compound having ACE inhibiting activity, a compound having AII antagonistic activity, a compound having Ca antagonistic activity, a compound having β-blocking activity, a compound having K channel opening activity, a compound having α-blocking activity, etc.); a compound having an activity to treat acute cerebral infarction; a compound having antiulcer activity (e.g., a compound having PPI inhibiting activity, a compound having H2 antagonistic activity, etc.); a compound having thrombolytic activity; a compound having an activity to treat diabetic complications (e.g., a compound having aldose reductase inhibiting activity, etc.); a compound having immunosuppressive activity; a compound having anti-tumor activity; radioisotope drug; and the like are preferably used, particularly, a compound having an activity to treat acute cerebral infarction; a compound having thrombolytic activity; a compound having anti-tumor activity; radioisotope drug; and the like are preferably used.

As used herein, for example, COX inhibiting activity means an activity to decrease COX activity, and may be, for example, an activity to inhibit COX activity or an activity to decrease COX expression. In the specification, COX inhibiting activity means, for example, presentation of clear COX inhibiting activity at a concentration of 10 µM or less *in vitro.* Specifically, it is preferred that COX inhibiting activity is showed at 10 µM or less of IC₅₀ value in Test Example 1 below.

In the present invention, the "compound having HSP inducing activity" is useful for decreasing tissue disorders caused by the "compound having an activity capable of causing a tissue disorder (e.g., compound having COX inhibiting activity, etc.)". In the specification, the "compound having HSP inducing activity" and the "compound having an activity capable of causing a tissue disorder" may be the same compound (a compound simultaneously having both activities) or compounds different from each other (two compounds are used together); however, the same compound is preferred in view of the burden on the patient who undergoes the administration, labor for preparation and the like. In addition, based on HSP inducing activity caused by the "compound having HSP inducing activity", not only a decrease in the side effects such as tissue disorder caused by the "compound having an activity capable of causing a tissue disorder*"*, but also an increase in the main activity of the "compound having an activity capable of causing a tissue disorder" (e.g., COX inhibiting activity etc.) are expected as a result of the clarification of medicinal properties.

In the present invention, the "compound having HSP inducing activity and an activity capable of causing a tissue disorder" is useful for decreasing tissue disorders caused by the "compound having an activity capable of causing a tissue disorder". In the specification, the "compound having HSP inducing activity and an activity capable of causing a tissue disorder" and the "compound having an activity capable of causing a tissue disorder" may be the same compound or different compounds, and preferably the same compound.

In the present invention, moreover, the "compound having HSP inducing activity and COX inhibiting activity" is useful for decreasing tissue disorders caused by the "compound having COX inhibiting activity". In the specification, the "compound having HSP inducing activity and COX inhibiting activity" and the "compound having COX inhibiting activity" may be the same compound or different compounds, and preferably the same compound.

When the "compound having HSP inducing activity and COX. inhibiting activity" and the "compound having COX inhibiting activity" are different compounds; preferred examples of the compound used as the "compound having COX inhibiting activity" include NSAIDs such as Diclofenac, Indomethacin, Aspirin, Ibuprofen, Ketoprophen, Piroxicam, Celecoxib, Rofecoxib, MK-663, Valdecoxib, SC-57666, JTE-522, S-2474,. SC-57666 and ML-3000.

When the "compound having HSP inducing activity and an activity capable of causing a tissue disorder" and the "compound having an activity capable of causing a tissue disorder" are different, and particularly when administration of the "compound having HSP inducing activity and an activity capable of causing a tissue disorder" is employed after a tissue disorder has been developed by the "compound having an activity capable of causing a tissue disorder", it is useful for decreasing progression of the tissue disorder caused by the "compound having an activity capable of causing a tissue disorder" and aggravation of the disease state.

Examples of the "compound having HSP inducing activity and COX inhibiting activity" are compounds represented by the formula (I) described in WO 01/72749: 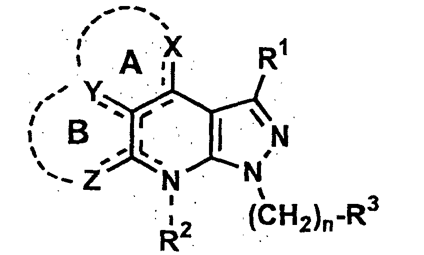 wherein R¹, is a hydrogen atom, a hydrocarbon group which may be substituted, an amino group which may-be substituted, a sulfur atom which may be substituted (-SR⁴ wherein R⁴ is a hydrogen atom, a hydrocarbon group which may be substituted, heterocyclic group which may.be substituted, etc.) or a carboxyl group which may be esterified or amidated; R² is unsubstituted, or a hydrogen atom or a hydrocarbon group which may be substituted; R³ is a heterocyclic group which may be substituted; X, Y and Z are, respectively, hydrogen, halogen, nitrile, a hydrocarbon group which may be substituted, a carboxyl group which may be esterified or amidated, an acyl group which may be substituted, -NR⁴ R⁵, an oxygen atom, -OR⁴, a sulfur atom, or -SR⁴ (R⁴ and R⁵ are, respectively, a hydrogen atom, a hydrocarbon group which may be substituted, or a heterocyclic group which may be substituted, or may bind to each other to form a cyclic amino group together with the nitrogen atom they bind with), or X and Y may bind to each other to form ring A, or Y and Z may bind to each other to form ring B; bond portions indicated by both solid and broken lines are, respectively, a single bond or a double bond, and bond portions indicated by a broken line are, respectively, a single bond or unsubstituted; ring A is a homocyclic or heterocyclic 5- to 7-membered ring which may be substituted; ring B is a homocyclic or heterocyclic 5- to 7-membered ring which may be substituted; and n is an integer of 0 or 1, or a salt thereof and the like. Preferable examples are a compound represented by the formula (Ia) : 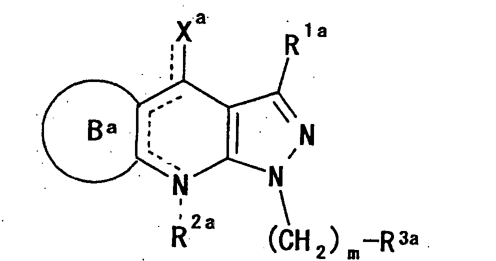 wherein R^{1a} is a hydrogen atom, a hydrocarbon group which may be substituted, or a carboxyl group which may be esterified or amidated; R^{2a} is unsubstituted, a hydrogen atom, or a hydrocarbon group which may be substituted; R^{3a} is a heterocyclic group which may be substituted; X^{a} is hydrogen, halogen, nitrile, a hydrocarbon group which may be substituted, a carboxyl group which may be esterified or amidated, an acyl group which may be substituted, -NR^{4a}R^{5a}, an oxygen atom, -OR^{4a}, a sulfur atom, or -SR^{4a} (R^{4a} and R^{5a} are, respectively, a hydrogen atom, a hydrocarbon group which may be substituted, or may bind to each other to form a cyclic amino group together with the nitrogen atom they bind with); bond portions indicated by solid and broken lines are, respectively, a single bond or a double bond, and bond portions indicated by a broken line are, respectively, a single bond or unsubstituted; ring B^{a} is a homocyclic or heterocyclic 5- to,7-membered ring which may be substituted; and m is an integer of 0 or 1, or a salt thereof and the like. Among them, more preferable examples are a compound represented by the formula (Ia'): 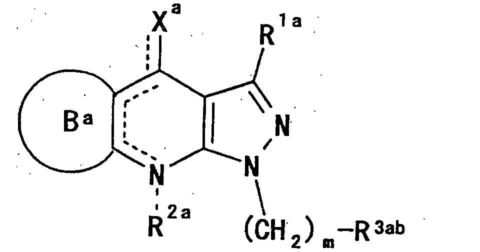 wherein R^{1a} is a hydrogen atom, a hydrocarbon group which may be substituted, or a carboxyl group which may be esterified or amidated; R^{2a} is unsubstituted, a hydrogen atom, or a hydrocarbon group which may be substituted; R^{3ab} is an unsaturated heterocyclic group having 2 or less nitrogen atoms as the hetero atoms which may be substituted, or an unsaturated monocyclic heterocyclic group having a nitrogen atom and a sulfur atom as the hetero atoms; X^{a} is hydrogen, halogen, nitrile and a hydrocarbon group which may be substituted, a carboxyl group which may be esterified or amidated, an acyl group which may be substituted; -NR^{4a}R^{5a}, an oxygen atom, -OR^{4a}, a sulfur atom, or -SR^{4a} (R^{4a} and R^{5a} are, respectively, a hydrogen atom, a hydrocarbon group which may be substituted, or may bind to each other to form a cyclic amino group together with the nitrogen atom they bind with); bond portions indicated by solid and broken lines are, respectively, a single bond or a double bond, and bond portions indicated by a broken line are, respectively, a single bond or unsubstituted; ring B^{a} is a homocyclic or heterocyclic 5- to 7-membered ring which may be substituted; and m is an integer of 0 or 1, or a salt thereof and the like. Concrete examples preferably used are 3-methyl-1-(2-pyridinyl)-6-trifluoromethyl-1,9-dihydro-4H-pyrazolo[3,4-b]quinoline-4-one (hereinafter abbreviated as Compound A), 6,7-difluoro-3-methyl-1-(2-pyridinyl)-1,9-dihydro-4H-pyrazolo(3,4-b]quirioline-4-one (hereinafter abbreviated as Compound B), 6-chloro-7-fluoro-3-methyl-1-(2-pyridinyl)-1,9-dihydro-4H-pyrazolo[3,4-b]quinoline-4-one, (hereinafter abbreviated as Compound C), 6-chloro-1- (,2-pyridinyl) -1, 9-dihydro-4H-pyrazolo[3,4-b]quinoline-4-one (hereinafter abbreviated as Compound D) or a salt thereof. These compounds can be also used as HSP inductors respectively.

Hereinafter the formula (I) is described in detail.

The "hydrocarbon groups" in the phrase "hydrocarbon group which may be substituted" used in the present specification, are, for example, an aliphatic hydrocarbon group, a saturated monocyclic hydrocarbon group, an aromatic hydrocarbon group, etc., preferably the group having 1 to 16 carbons. More. specifically, examples of the hydrocarbon group are alkyl groups, alkenyl groups, alkynyl groups, cycloalkyl groups, aryl groups, etc.

Preferred examples of the "alkyl group" include a lower alkyl group or the like, more specifically, C₁₋₆ alkyl groups such as methyl, ethyl, propyl, isopropyl, butyl, isobutyl, sec-butyl, tert-butyl, pentyl, hexyl, etc.

Preferred examples of the "alkenyl group" are lower alkenyl groups or the like, more specifically C₂₋₆ alkenyl groups such as vinyl, 1-propenyl, allyl, isopropenyl, butenyl, isobutenyl, etc.

Preferred examples of the "alkynyl group" are lower alkynyl groups or the like, more specifically a C₂₋₆ alkynyl group such as ethynyl, propargyl and 1-propynyl, etc.

Preferred examples of the "cycloalkyl group" are lower cycloalkyl groups or the like, more specifically C₃-₆ cycloalkyl groups such as cyclopropyl, cyclobutyl, cyclopentyl, cyclohexyl, etc.

Preferred examples of the "aryl group" are C₆₋₁₄ aryl groups or the like, such as phenyl, 1-naphthyl, 2-naphthyl, biphenylyl, 2-indenyl, 2-anthryl, etc. more preferably phenyl group, etc.

Examples of the substituent of the "hydrocarbon group" in the "hydrocarbon group which may be substituted" include halogen atoms (e.g., fluorine, chlorine, bromine, iodine, etc.,); a nitro group; a cyano group; a hydroxyl group; lower alkyl groups which may be halogenated (e.g. , C₁₋₆ alkyl groups which may be halogenated such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, isobutyl, sec-butyl, tert-butyl, 4,4,4-trifluorobutyl, pentyl, isopentyl, neopentyl, 5,5,5-trifluoropentyl, hexyl, 6,6,6-trifluorohexyl, etc.); lower alkoxy groups (e.g., C₁₋₆ alkoxy groups such as methoxy, ethoxy, propoxy, isopropoxy, cyclopropoxy, butoxy, isobutoxy, cyclobutoxy, pentyloxy, cyclopentyloxy, hexyloxy, cyclohexyloxy, etc.); an amino group; mono-lower alkylamino groups (e.g., mono-C₁₋₆ alkylamino groups such as methylamino, ethylamino, etc.); di-lower alkylamino groups (e.g., di-C₁₋₆ alkylamino groups such as dimethylamino, diethylamino, etc.); a carboxyl group; lower alkyl carbonyl groups (e.g., C₁₋₆ alkylcarbonyl groups such as acetyl, propionyl, etc.); lower alkoxycarbonyl groups (e. g., C₁₋₆ alkoxycarbonyl groups such as methoxycarbonyl, ethoxycarbonyl, propoxycarbonyl, butoxycarbonyl, etc.); a carbamoyl group; mono-lower alkylcarbamoyl groups (e.g., mono-C₁₋₆ alkylcarbamoyl groups such as methylcarbamoyl, ethylcarbamoyl, etc.); di-lower alkylcarbamoyl groups (e.g., di-C₁₋₆ alkylcarbamoyl groups such as dimethylcarbamoyl, diethylcarbamoyl, etc.); arylcarbamoyl groups (e.g., C₆₋₁₀ arylcarbamoyl groups such as phenylcarbamoyl, naphthylcarbamoyl, etc.); aryl groups (e.g., C₆₋₁₀ aryl groups such as phenyl, naphthyl, etc.); aryloxy groups (e.g. , C₆₋₁₀ aryloxy group such as phenyloxy, naphthyloxy, etc.); and lower alkylcarbonylamino groups which may be halogenated (e.g., C₁₋₆ alkylcarbonylamino groups which may be halogenated such as acetylamino, trifluoroacetylamino, etc.).

The "hydrocarbon group" of the "hydrocarbon group which may be substituted" may have 1 to 5, preferably 1 to 3, substituents described above at places where the substitution is possible, and in the case of the hydrocarbon group having 2 or more substituents, the substituents may be same or different.

Examples of the term the "heterocyclic group" in the "heterocyclic group which may be substituted" of the present specification include 5- to 14-membered, preferably 5- to 10-membered, (monocyclic to tricyclic, preferably monocyclic to bicyclic) heterocyclic groups having 1 to 4, preferably 1 to 3 atoms which are 1 to 2 hetero atoms selected from the group consisting of nitrogen, oxygen and sulfur atoms as well as carbon atoms. More specifically, examples of the heterocyclic group include 5-membered ring groups having 1 to 4 hetero atoms selected from the group consisting of oxygen, sulfur and nitrogen atoms as well as carbon atoms such as 2- or 3-thienyl, 3-furyl, 1-, 2- or 3-pyrrolyl, 1-, 2- or 3-pyrrolidinyl, 2-, 4- or 5-oxazolyl, 3-, 4- or 5-isooxazolyl, 2-, 4- or 5-thiazolyl, 3-, 4- or 5-isothiazolyl, 3-, 4- or 5-pyrazolyl, 2-, 3- or 4-pyrazolidinyl, 2-, 4- or 5-imidazolyl, 1,2,3-triazolyl, 1,2,4-triazolyl, 1H- or 2H-tetrazolyl, etc.; 6-membered ring groups having 1 to 4 hetero atoms selected from the group consisting of oxygen, sulfur and nitrogen atoms as well as carbon atoms such as 2-, 3- or 4-pyridinyl, N-oxide-2-, 3- or 4-pyridinyl, 2-, 4- or 5-pyrimidinyl, N-oxide-2-, 4- or 5-pyrimidinyl, thiomorpholinyl, morpholinyl, piperidino, 2-, 3- or 4-piperidyl, thiopyranyl, 1,4-oxazinyl, 1,4-thiazinyl, 1,3-thiazinyl, piperazinyl, triazinyl, 3- or 4-pyridazinyl, pyrazinyl, N-oxide-3- or 4-pyridazinyl, etc.; and bicyclic or tricyclic condensed ring groups having 1 to 4 hetero atoms selected from the group consisting of oxygen, sulfur and nitrogen atoms as well as carbon atoms (preferably, groups which are formed by the condensation of the 5 or 6-membered ring groups described above and 1 or 2 5- or 6-membered rings optionally having 1 to 4 hetero atoms selected from the group consisting of oxygen, sulfur and nitrogen atoms as well as carbon atoms) such as indolyl, benzofuryl, benzoxazolyl, benzimidazolyl, quinolinyl, isoquinolinyl, phthalazinyl, quinazolinyl, quinoxalinyl, indolizinyl, quinolizinyl, 1,8-naphthyridinyl, dibenzofuranyl, carbazolyl, acridinyl, phenanthridinyl, chromanyl, phenothiazinyl, phenoxazinyl, etc. The heterocyclic group is preferably a 5-to 7-membered (preferably 5- or 6-membered) heterocyclic ring having 1 to 3 hetero atoms selected from the group consisting of oxygen, sulfur and nitrogen atoms as well as carbon atoms.

Examples of the substituent of the "heterocyclic group" of the "heterocyclic group which may be substituted" include halogen atoms (e.g., fluorine, chlorine, bromine, iodine, etc.); lower alkyl groups (e.g., C₁₋₆ alkyl groups such as methyl, ethyl, propyl, isopropyl, butyl, isobutyl, sec-butyl, tert-butyl, pentyl, hexyl, etc); cycloalkyl groups (e.g., C₃₋₆ cycloalkyl groups such as cyclopropyl, cyclobutyl, cyclopentyl, cyclohexyl, etc.); lower alkynyl groups (e.g., C₂₋₆ alkynyl groups such as ethynyl, 1-propynyl, propargyl, etc.); lower alkenyl groups (e.g., C₂₋₆ alkenyl groups such as vinyl, allyl, isopropenyl, butenyl, isobutenyl, etc.); aralkyl groups (e.g., C₇₋₁₁ aralkyl groups such as benzyl, a-methylbenzyl, phenethyl, etc.); aryl groups (e.g., C₆₋₁₀ aryl groups such as phenyl, naphthyl, etc. preferably phenyl group); lower alkoxy groups (e.g., C₁₋₆ alkoxy groups such as methoxy, ethoxy; propoxy, isopropoxy, butoxy, isobutoxy, sec-butoxy, tert-butoxy, etc.) ; aryloxy groups (e.g., C₆₋₁₀ aryloxy groups such as phenoxy, etc.); lower alkanoyl groups (e.g., C₁₋₆ alkanoyl groups such as formyl, acetyl, propionyl, butyryl, isobutyryl); arylcarbonyl (e.g., C₆₋₁₀ arylcarbonyl groups such as benzoyl, naphthoyl, etc.); lower alkanoyloxy groups (e.g., C₁₋₆ alkanoyloxy groups such as formyloxy, acetyloxy, propionyloxy, butyryloxy, isobutyryloxy, etc.); arylcarbonyloxy groups (e.g., C₆₋₁₀ arylcarbonyloxy groups such as benzoyloxy, naphthoyloxy, etc.); a carboxyl group; lower alkoxycarbonyl groups (e.g., .C₁₋₆ alkoxy-carbonyl groups such as methoxycarbonyl, ethoxycarbonyl, propoxycarbonyl; isopropoxycarbonyl, butoxycarbonyl, isobutoxycarbonyl, tert-butoxycarbonyl, etc.); aralkyloxycarbonyl groups (e.g., C₇₋₁₁ aralkyloxycarbonyl groups such as benzyloxycarbonyl, etc.) ; a carbamoyl group; mono-, di- or tri-halogeno-lower alkyl groups (e.g., mono-, di- or tri-halogeno-C₁₋₄ alkyl groups such as chloromethyl, dichloromethyl, trifluoromethyl, 2,2,2-trifluoroethyl, etc.); an oxo group; an amidino group; an imino group; an amino group; mono-lower alkylamino groups (e.g., mono-C₁₋₄ alkylamino groups such as methylamino, ethylamino, propylamino, isopropylamino, butylamino, etc.); di-lower alkylamino groups (e.g., di-C₁₋₄ alkylamino groups such as dimethylamino, diethylamino, dipropylamino, diisopropylamino, dibutylamino, etc.) ; 3- to 6-membered cyclic amino groups optionally having 1 to 3 hetero atoms selected from the group consisting of oxygen, sulfur, and nitrogen atoms as well as carbon atoms and a nitrogen atom (e.g., 3- to 6-membered cyclic amino groups such as aziridinyl, azetidinyl, pyrrolidinyl, pyrrolinyl, pyrrolyl, imidazolyl, pyrazolyl, imidazolidinyl, piperidinyl, morpholinyl, dihydropyridinyl, pyridinyl, N-methylpiperazinyl, N-ethylpiperazinyl), alkylenedioxy groups (e.g., C₁₋₃ alkylenedioxy groups such as methylenedioxy, ethylenedioxy, etc.); a hydroxyl group; a nitro group; a cyano group; a mercapto group; a sulfo group, a sulfino group, a phosphono group, a sulfamoyl group, mono-alkylsulfamoyl groups (e.g., mono-C₁₋₆ alkylsulfamoyl groups such as N-methylsulfamoyl, N-ethylsulfamoyl, N-propylsulfamoyl, N-isopropylsulfamoyl, N-butylsulfamoyl; etc.); di-alkylsulfamoyl groups (e.g., di-C₁₋₆ alkylsulfamoyl groups such as N,N-dimethylsulfamoyl, N,N-diethylsulfamoyl, N,N-dipropylsulfamoyl, N,N-dibutylsulfamoyl, etc.); alkylthio groups (e.g., C₁₋₆ alkylthio groups such as methylthio, ethylthio, propylthio, isopropylthio, butylthio, sec-butylthio, tert-butylthio, etc.); arylthio groups (e.g., C₆₋₁₀ arylthio groups such as phenylthio, naphthylthio, etc.); lower alkylsulfinyl groups (e.g., C₁₋₆ alkylsulfinyl groups such as methylsulfinyl, ethylsulfinyl, propylsulfinyl, isopropylsulfinyl, butylsulfinyl, etc.); arylsulfinyl groups (e.g., C₆₋₁₀ arylsulfinyl groups such as phenylsulfinyl, naphthylsulfinyl, etc.); lower alkylsulfonyl groups (e.g., C₁₋₆ alkylsulfonyl groups such as methylsulfonyl, ethylsulfonyl, propylsulfonyl, isopropylsulfonyl, butylsulfonyl, etc.); and arylsulfonyl groups (e.g., C₆₋₁₀ arylsulfonyl groups such as phenylsulfonyl, naphthylsulfonyl, etc.).

The "heterocyclic group" of the "heterocyclic. group which may be substituted" may have 1 to 5, preferably 1 to 3, substituent described above at the positions of the heterocyclic ring where the substitution is possible, and in the case of the ring having 2 or more substituents, the substituents may be same or different.

An example of the term the "amino group which may be substituted" of the present specification, is an amino group which may have 1 or 2 "hydrocarbon groups.which may be substituted" described above. Preferred examples of the substituent of the "amino group" include C₁₋₆ alkyl groups which may be substituted, and C₆₋₁₀ aryl groups which may be substituted. The substituents which the "C₁₋₆ alkyl group" and the "C₆₋₁₀ aryl group" may have are similar to the substituents of the "hydrocarbon groups" described above.

Examples of the term "lower alkoxy group" in the phrase "lower alkoxy group which may be substituted" of the present specification include C₁₋₆ alkoxy groups such as methoxy, ethoxy, propoxy, isopropoxy, butoxy, isobutoxy, sec-butoxy, tert-butoxy, etc. and the lower alkoxy group may have 1 to 3 substituents similar to those of the "hydrocarbon group" described above.

Examples of the term "benzene ring which may be substituted" of the present specification include benzene rings optionally having, at the position where the substitution is possible, 1 to 3 (preferably 1 or 2) same or different substituents selected from the group of substituents consisting of halogen atoms (e.g., fluorine, chlorine, bromine, iodine; etc.); hydrocarbon groups which may be substituted; amino groups which may be substituted; amide groups (e.g., C₁₋₆ acylamino groups such as acetamide, etc., preferably C₁₋₆ alkanoylamino groups, etc.) ; lower alkoxy groups which may be substituted; lower alkylenedioxy groups (e.g., C₁₋₆ alkylenedioxy groups such as methylenedioxy, ethylenedioxy); and substituents similar to those which the "heterocyclic group" in the "heterocycle group which may be substituted" may have.

Examples of the term "hydrocarbon group which may be substituted", the "amino group which may be substituted" and the "lower alkoxy group which may be substituted" are similar to those described above in detail. In the case of the "hydrocarbon group", the "amino group" and the "lower alkoxy group" having 2 or more substituents, the substituents may be same or different.

The "benzene ring which may be substituted" includes preferably a benzene ring which may be substituted by 1 to 3 substituents selected, for example, from the group consisting of halogen atoms (e.g., fluorine, chlorine, etc.), C₁₋₆ alkyl groups (e.g., methyl, ethyl, etc.), and mono-C₁₋₆ alkylamino-groups.

The terrn "sulfur atom which may be substituted" of the present invention represents a group represented by the formula: -SR⁴ wherein R⁴ is a hydrogen atom, a hydrocarbon group which may be substituted or a heterocyclic group which may be substituted.

The term "carboxyl group which may be esterified" is a group represented by -COOR⁶. Here, R⁶ is a hydrogen atom or a hydrocarbon group which may be substituted. The "carboxyl group which may be amidated" is a group represented by-CONR⁷R⁸. Here, R⁷ and R⁸ are, respectively, a hydrogen atom, a hydrocarbon group which may be substituted, or may bind to each other to form a cyclic amino group together with the nitrogen atom they bind with.

The term "acyl group which may be substituted" is a group represented by -COR⁹, -SOR⁹ or -SO₂R⁹. Here, R⁹ is the "hydrocarbon group which may be substituted*"* or the "heterocycle group which may be substituted", both of which are described above.

The examples of the "cyclic amino group formed by both of R⁴ and R⁵ together with the nitrogen atom they bind with" in the definition of -NR⁴R⁵ and the "cyclic amino group formed by both of R⁷ and R⁸ together with the nitrogen atom they bind with" in the definition of -CONR⁷R⁸ are 3- to 6-membered cyclic amino groups optionally having 1 to 3 hetero atoms selected from the group consisting of oxygen, sulfur, and nitrogen atoms as well as carbon atoms and a nitrogen atom (e.g., 3- to 6-membered cyclic amino groups such as aziridinyl, azetidinyl, pyrrolidinyl, pyrrolinyl, pyrrolyl, imidazolyl, pyrazolyl, imidazolidinyl, piperidinyl, morpholinyl, dihydropyridinyl, pyridinyl, N-methylpiperazinyl, N-ethylpiperazinyl, etc.), etc.

Preferably, the heterocyclic group of R³ is a nitrogen-containing aromatic heterocyclic group, especially, a 6-membered nitrogen-containing aromatic heterocycle group, for example, a pyridine ring. The substituents thereof are those of the "heterocyclic group which may be substituted" described above, and R³ may be a quinoline ring which is formed by the condensation of the nitrogen-containing heterocyclic group and a benzene ring.

X is preferably a hydrogen atom, an oxygen atom, -OR⁴ (R⁴ is a hydrogen atom or a hydrocarbon group which may be substituted) or a hydrocarbon group which may be substituted.

Y is preferably a hydrogen atom, a hydrocarbon group which may be substituted, -COR⁹ or -COOR⁶, more preferably -COR⁹ or -COOR⁶. Z is preferably a hydrogen atom, an oxygen atom,-OR⁴ or a hydrocarbon group which may be substituted (herein, R⁴ is a hydrogen atom or a hydrocarbon group which may be substituted).

Examples of the homocyclic ring of the "5- to 7-membered homocyclic or heterocyclic ring which may be substituted" represented by ring A or ring B include cyclopentane, cyclohexane, cycloheptane, cyclopentene, cyclopentadiene, cyclohexene, cyclohexadiene, benzene, cycloheptene, cycloheptadiene, etc., preferably benzene, cyclopentane, cyclohexane, cycloheptane, and most preferably benzene.

Examples of the heterocyclic ring of the "5- to 7-membered homocyclic or heterocyclic ring which may be substituted" represented by ring A or ring B include aromatic heterocyclic rings such as furan, thiophene, pyrrole, oxazole, isoxazole, thiazole, isothiazole, imidazole, pyrazole, oxadiazole, furazan, thiadiazole, triazole, pyridine, pyridazine, pyrimidine, pyrazine, triazine, etc.; non-aromatic heterocyclic rings such as azetidine, oxetane, pyrrolidine, piperidine, tetrahydropyran, morpholine, thiomorpholine, piperazine, etc.; and non-aromatic heterocyclic rings wherein a part of or all double bonds in the aromatic heterocyclic rings are saturated.

The substituents of the "5- to 7-membered homocyclic or heterocyclic ring which may be substituted" represented by ring A or ring B are those which the "heterocyclic ring which may be substituted" described above may have, and the number of substitution is also same as that of the heterocyclic ring.

n is preferably 0.

Preferably, the heterocyclic group of R^{3a} is an unsaturated heterocyclic group containing 2 or less of nitrogen atoms as hetero atoms or an unsaturated monocyclic heterocyclic group containing a nitrogen atom and a sulfur atom as hetero atoms. More preferably the heterocyclic group. of R^{3a} is a nitrogen-containing aromatic heterocyclic group, especially, a 6-membered nitrogen-containing aromatic heterocycle group, for example, a pyridine ring. The substituents thereof are those of the "heterocyclic group which may be substituted" described above, and R^{3a} may be a quinoline ring which is formed by the condensation of the nitrogen-containing heterocyclic group and a benzene ring. The unsaturated heterocyclic group containing 2 or less of nitrogen atoms as hetero atoms or an unsaturated monocyclic heterocyclic group containing a nitrogen atom and a sulfur atom as hetero atoms of R^{3ab} is preferably a nitrogen-containing aromatic heterocyclic group, especially, a 6-membered nitrogen-containing aromatic heterocycle group, for example, a pyridine ring. The substituents thereof are those of the "heterocyclic group which may be substituted" described above, and R^{3ab} may be a quinoline ring which is formed by the condensation of the nitrogen-containing heterocyclic group and a benzene ring.

In addition, X^{a} is preferably an oxygen atom or -OR^{4a} wherein R^{4a} is a hydrogen atom or a hydrocarbon group which may be substituted. Ring B and ring B^{a} are preferably benzene ring which may be substituted. Especially the compound (Ia) or (Ia') wherein R^{3a} or R^{3ab} is a nitrogen containing aromatic heterocyclic group and ring B^{a} is a benzene ring which may be substituted.

m is preferably 0.

Preferred specific examples of the compound (I), (Ia) and (Ia') of the present invention are followings:
Compound (I) wherein R² is unsubstituted or a hydrogen atom and X and Y may bind to each other to form ring A;
Compound (I) wherein R³ is an unsaturated heterocyclic group containing a nitrogen atom as a heteroatom and n is 0;
Compound (I) wherein Y and Z may bind to each other to form ring B and ring B is a homocyclic or heterocyclic 5- to 7-membered ring which may be substituted;
Compound (I) wherein hydrocarbon groups of R¹ and R² are respectively an aliphatic hydrocarbon group, a monocyclic saturated hydrocarbon group or an aromatic hydrocarbon group;
Compound (I) wherein hydrocarbon groups of R¹ and R² are respectively an alkyl group, an alkenyl group; an alkynyl group, a cycloalkyl group or an aryl group, each of the groups having 1 to 16 carbons;
Compound (I) wherein the homocyclic or heterocyclic ring of ring A or ring B is a monocyclic saturated hydrocarbon, benzene ring, pyridine ring or thiophene ring;
Compound (I) wherein X is a hydrogen atom, an oxygen atom, -OR⁴ (R⁴ has the same meaning defined above) or a hydrocarbon group which may be substituted;
Compound (I) wherein Y is -COR⁹ or -COOR⁶ (R⁶ and R⁹ have the same meaning defined above);
Compound (I) wherein Z is a hydrogen atom, an oxygen atom, -OR⁴ (R⁴ has the same meaning defined above) or a hydrocarbon group which may be substituted;
Compound (Ia) wherein the hydrocarbon groups of R^{1a} _{and} R^{2a} are respectively an aliphatic hydrocarbon group, a saturated monocyclic hydrocarbon group or an aromatic. hydrocarbon group;
Compound (Ia) wherein the hydrocarbon groups of R^{1a} and R^{2a} are respectively an alkyl group, an alkenyl group, an alkynyl group, a cycloalkyl group or an aryl group, each of the groups having 1 to 16 carbons;
Compound (Ia) wherein the homocyclic or heterocyclic ring of ring B^{a} is a monocyclic saturated hydrocarbon, benzene ring, pyridine ring or thiophene ring;
Compound (Ia) wherein X^{a} is a hydrogen atom, an oxygen atom, -OR^{4a} (R^{4a} has the same meaning defined above) or a hydrocarbon group which may be substituted;
Compound (Ia' ) wherein R^{1a} and R^{2a} are respectively an alkyl group, an alkenyl group, an alkynyl group, a cycloalkyl group or an aryl group, each of the groups having 1 to 16 carbons;
Compound (Ia') wherein R^{3ab} is pyridinyl which may be substituted by (1) a halogen atom, (2) a lower alkyl group, (3) a cycloalkyl group, (4) a lower alkynyl group, (5) a lower alkenyl group, (6) an aralkyl group, (7) an aryl group, (8) a lower alkoxy group, (9) an aryloxy group, (10) a lower alkanoyl group, (11) an arylcarbonyl group, (12) a lower alkanoyloxy group, (13) an arylcarbonyloxy group, (14) a carboxyl group, (15) a lower alkoxycarbonyl group, (16) an aralkyloxycarbonyl group, (17) a carbamoyl group, (18) a mono-, di- or tri-halogeno-lower alkyl group, (19) an amidino group, (20) an amino group, (21) a mono-lower alkylamino group, (22) a di-lower alkylamino group, (23) a 3- to 6-membered cyclic amino group which may have 1 to 3 hetero atoms selected from oxygen atom, sulfur atom and nitrogen atom in addition to carbon atoms and a nitrogen atom, (24) an alkylenedioxy group, (25) a hydroxyl group, (26) a nitro group, (27) a cyano group, (28) a mercapto group, (29) a sulfo group, (30) a sulfino group, (31) a phosphono group, (32) a sulfamoyl group, (33) a mono-alkylsulfamoyl group, (34) a di-alkylsulfamoyl group, (35) . an alkylthio group, (36) an arylthio group, (37) a lower alkylsulfinyl group, (38) an arylsulfinyl group, (39) a lower alkylsulfonyl group or (40) an arylsulfonyl group;
Compound (Ia') wherein X^{a} is an oxygen atom, -OR^{4a} (R^{4a} is a hydrogen atom or a hydrocarbon group which may be substituted by (1) a halogen atom, (2) a nitro group, (3) a cyano group, (4) a hydroxyl group, (5) a lower alkyl group which may be halogenated, (6) a lower alkoxy group, (7) an amino group, (8) a mono-lower alkylamino group, (9) a di-lower alkylamino group, (10) a carboxyl group, (12) a lower alkoxy-carbonyl group, (13) a carbamoyl group, (14) a mono-lower alkylcarbamoyl group, (15) a di-lower alkylcarbamoyl group, (16) an arylcarbamoyl group, (17) an aryl group, (18) an aryloxy group or (19) a lower alkylcarbonylamino group which may be halogenated);
Compound (Ia') wherein R^{3ab} is a nitrogen-containing aromatic heterocyclic group and ring B^{a} is a benzene ring which may be substituted by (1) a halogen atom, (2) a hydrocarbon group which may be substituted, (3) an amino group which may be substituted, (4) a lower alkoxy group which may be substituted, (5) a lower alkylenedioxy group, (6) an aryloxy group, (7) an lower alkanoyl group, (8) an arylcarbonyl group, (9) a lower alkanoyloxy group, (10) an arylcarbonyloxy group, (11) a carboxyl group, (12) a lower alkoxycarbonyl group, (13) an aralkyloxycarbonyl, (14) carbamoyl group, (15) mono-, di-or tri-halogeno-lower alkyl group, (16) an amidino group, (17) an amino group, (18) a mono-lower alkylamino group, (19) di-lower alkylamino group, (20) a 3- to 6-membered cyclic amino group which may have 1 to 3 hetero atoms selected from oxygen atom, sulfur atom and nitrogen atom in addition to carbon atoms and a nitrogen atom, (21) an alkylenedioxy group, (22) a hydroxyl group, (23) a nitro group, (24) a cyano group, (25) a mercapto group, (26) a sulfo group, (27) a sulfino group, (28) a phosphono group, (29) a sulfamoyl group, (30) mono-alkylsulfamoyl group, (31) di-alkylsulfamoyl group, (32) an alkylsulfanyl group, (33) arylsuifanyl group, (34) a lower alkylsulfinyl group, (35) an arylsulfinyl group, (36) a lower alkylsulfonyl group or (37) an arylsulfonyl group;

Especially preferable compounds of Compound (Ia) and (Ia') are, for example, 6,7-difluoro-3-methyl-1-(2-pyridinyl)-1,9-dihydro-4H-pyrazolo[3,4-b]quinolin-4-one, 3-methyl-1- (2-pyridinyl)-6-trifluoromethyl-1,9-dihydro-4H-pyrazolo[3,4-b]quinolin-4-one, 6-fluoro-3-methyl-1-(2-pyridinyl)-1,9-dihydro-4H-pyrazolo[3,4-b]quinolin-4-one, 7-fluoro-3-methyl-1-(2-pyridinyl)-1,9-dihydro-4H-pyrazolo[3,4-b]quinolin-4-one, 3-ethyl-6,7-difluoro-1-(2-pyridinyl)-1,9-dihydro-4H-pyrazolo[3,4-b]quinolin-4-one, 6,7-difluoro-3-methyl-1-(3-pyridinyl)-1,9-dihydro-4H-pyrazolo[3,4-b]quinolin-4-one, 6,7-difluoro-3-methyl-1-(6-methyl-2-pyridinyl)-1,9-dihydro-4H-pyrazolo[3,4-b]quinolin-4-one, 6,7-difluoro-3-methyl-1-(6-phenyl-2-pyridinyl)-1,9-dihydro-4H-pyrazolo[3,4-b]quinolin-4-one, 5-fluoro-3-methyl-1-(2-pyridinyl)-1,9-dihydro-4H-pyrazolo[3,4-b]quinolin-4-one, 1-(2-pyridinyl)-1,9-dihydro-4H-pyrazolo[3,4-b]quinolin-4-one, 6-chloro-7-fluoro-3-methyl-1-(2-pyridinyl)-1,9-dihydro-4H-pyrazolo[3,4-b]quinolin-4-one and 6-chloro-1-(2-pyridinyl)-1,9-dihydro-4H-pyrazolo[3,4-b]quinolin-4-one, etc.

As the salts of the compound (I), (Ia) and (Ia') of the present invention, for example, pharmaceutically acceptable salts are used. Examples of the salt include salts with inorganic bases, salts with organic bases, salts with inorganic acids, salts with organic acids, salts with basic or acidic amino acids, etc. Suitable examples of the salt with an inorganic base include alkali metal salts such as sodium salt and potassium salt, alkali-earth metal salts such as calcium salt and magnesium salt, aluminum salt, ammonium salt and the like. Preferred examples of the salt with an organic base include salts with trimethylamine,.triethylamine, pyridine, picoline, 2,6-lutidine, ethanolamine, diethanolamine, triethanolamine, cyclohexylamine, dicyclohexylamine, N,N-dibenzylethylene diamine, etc. Preferred examples of the salt with an inorganic acid include salts with hydrochloric acid, hydrobromic acid, nitric acid, sulfuric acid, phosphoric acid, etc. Preferred examples of the salt with an organic acid include salts such as formate, acetate, trifluoroacetate, phthalate, fumarate, oxalate, tartrate, maleate, citrate, succinate, malate, methanesulfonate, benzenesulfonate, p-toluenesulfonate, etc. Preferred examples of the salt with a basic amino acid include salts with arginine, lysine, ornithine, etc., and examples of the salt with an acidic amino acid include salts with aspartic acid, glutamic acid, etc.

The salt is preferably a pharmaceutically acceptable salt, and in the case of the compound (I), (Ia) or (Ia') having a basic functional group, preferred examples of the salt include salts with inorganic acids such as hydrochloride, hydrobromide, nitrate, sulfate, phosphate, and salts with organic acids such as acetate, phthalate, fumarate, tartrate, maleate, citrate, succinate, methanesulfonate, p-toluenesulfonate, and in the case of the salt having an acidic functional group, examples of the salt include alkali metal salts such as sodium salt and potassium salt, alkali-earth metal salts such as calcium salt and magnesium salt, and ammonium salt.

In addition, the compounds (I), (Ia) and (Ia') may be hydrate or non-hydrate. Examples of such hydrate include 1.0 hydrate, 1.5 hydrate, 2.0 hydrate and the like.

The "compound having HSP inducing activity", the "compound having an activity capable of causing a tissue disorder", the "compound having HSP inducing activity and an activity capable of causing a tissue disorder", the "compound having COX inhibiting activity" and the "compound having HSP inducing activity and COX inhibiting activity" used in the present invention may form salts, and such salts include salts similar to those of the compounds (I), (Ia) and (Ia').

The compounds (I), (Ia) and (Ia') can be prepared, for example, in accordance with the methods.described in WO01/72749.

In the present invention, the "compound having COX inhibiting activity" and the "compound having HSP inducing activity and COX inhibiting activity" are not limited to those illustrated above, and include any compound as far as it has such activity. Additionally, the COX inhibiting activity may be COX-2 selective inhibiting activity.

The dose of the pharmaceutical composition of the present invention may be any as long as it is within the range of effective amounts of the "compound having HSP inducing activity", the "compound having an activity capable of causing a tissue disorder", the "compound having HSP inducing activity and an activity capable of causing a tissue disorder", the "compound having COX inhibiting activity" and the "compound having HSP inducing activity and COX inhibiting activity*"*.

The pharmaceutical composition of the present invention is orally administered to, for example, adult patient (body weight 60 kg) suffering from rheumatoid arthritis or osteoarthritis as the "compound having HSP inducing activity", the "compound having an activity capable of causing a tissue disorder", the "compound having HSP inducing activity and an activity capable of causing a tissue disorder", the "compound having COX inhibiting activity" or the "compound having HSP inducing activity and COX inhibiting activity" (preferably, the "compound having HSP inducing activity and COX inhibiting activity"). Its dose is about 0.1 to about 600 mg/day, preferably, about 1 to about 300 mg/day. In such dose, the compounds may be administered once daily or in 2 or 3 divided portions per day.

The pharmaceutical composition of the present invention shows low toxicity, and can be used as an agent for the prophylaxis and/or treatment of various diseases described below in mammals (e.g., human, mouse, rat, rabbit, dog, cat, bovine, horse, pig, monkey, etc).

The pharmaceutical compositions of the present invention may comprise a pharmaceutically acceptable carrier. Examples of such carrier include various organic or inorganic carriers commonly used for such formulations, for example, excipients, lubricants, binders, disintegrants, etc., for solid formulations, and solvents, solubilizing agents, suspending agents, isotonic agents, buffer agents, soothing agents, etc., for liquid formulations. Additionally, other additives may also be used, if desired, such as antiseptic substances, antioxidants, coloring agents and sweeteners. When the "compound having HSP inducing activity" and the "compound having an activity capable of causing a tissue disorder (e.g., the compound having COX inhibiting activity)" are different substances, the composition of the present invention may be a single formulation comprising both compounds (mixture) or may be two different formulations comprising any one of the compounds. In latter case, the dosage forms of the formulations are not necessarily the same. For these formulations, the dosage forms are appropriately selected depending on each active ingredient, which is usually used in medical practice.

Preferred examples of the excipient include lactose, saccharose, D-mannitol, D-sorbitol, starch, pregelatinized starch, dextrin, crystalline cellulose, low-substituted hydroxypropylcellulose, carboxymethylcellulose sodium, gum arabic, dextrin, pullulan, light anhydrous silicic acid, synthetic aluminum silicate, magnesium aluminometasilicate, and the like.

Preferred examples of the lubricant include magnesium stearate, calcium stearate, talc, colloidal silica, and the like.

Preferred examples of the binder include, for example, pregelatinized starch, sucrose, gelatin, gum arabic, methylcellulose, carboxymethylcellulose, carboxymethylcellulose sodium, crystalline cellulose, saccharose, D-mannitol, trehalose, dextrin, pullulan, hydroxypropylcellulose, hydroxypropylmethylcelulose, polyvinylpyrrolidone, and the like.

Preferred examples of the disintegrant include lactose, saccharose, starch, carboxymethylcellulose, carboxymethylcellulose calcium, sodium croscarmellose, carboxymethylstarch sodium, light anhydrous silicic acid, low-substituted hydroxypropylcellulose, and the like.

Preferred examples of the solvent include water for injection, physiological saline, Ringer's solution, alcohol, propyleneglycol, polyethyleneglycol, sesame oil, corn oil, olive oil, cottonseed oil and the like.

Preferred examples of the solubilizing agent include polyethyleneglycol, propyleneglycol, D-mannitol, trehalose, benzyl benzoate, ethanol, trisaminomethane, cholesterol, triethanolamine, sodium carbonate, sodium citrate, sodium salicylate, sodium acetate, and the like.

Preferred examples of the suspending agent include surfactants such as stearyltriethanolamine, sodium laurylsulfate, laurylaminopropionate, lecithin, benzalkonium chloride, benzethonium chloride, glycerol monostearate and the like; hydrophilic polymers such as polyvinylalcohol, polyvinylpyrrolidone, carboxymethylcellulose sodium, methylcellulose, hydroxymethylcellulose, hydroxyethylcellulose, hydroxypropylcellulose and the like; polysorbates; polyoxyethylene-hydrogenated castor oil and the like.

Preferred examples of the isotonic agent include sodium chloride, glycerin, D-mannitol, D-sorbitol, glycerol and the like.

Preferred examples of the buffer agent include buffer solutions of the salts such as phosphate, acetate, carbonate, citrate and the like.

Preferred examples of the soothing agent include benzyl alcohol, and the like.

Examples of the antiseptic substance include paraoxybenzoate esters, chlorobutanol, benzyl alcohol, phenethyl alcohol, dehydroacetic acid, sorbic acid, and the like.

Preferred examples of the antioxidant include sulfites, ascorbic acid and the like.

Preferred example of coloring agents include water-soluble, edible tar dyes (e.g., Food Colors such as Food Color Red No.,2 and No.3, Food Color Yellow No.4 and No. 5, Food Color Blue No.1 and No. 2), insoluble lake dyes (e.g., Aluminum salts of water-soluble, edible tar dyes described above, etc. ) , natural dyes (e. g., β-carotene, chlorophyll, iron oxide red, etc.), and the like.

Preferred examples of the sweetener include saccharin sodium, dipotassium glycyrrhizinate, aspartame, stevia and the like.

The dosage form of the pharmaceutical composition of the present invention include oral preparations such as tablets, capsules (including soft capsule and microcapsule), granules, powders, syrups, emulsions, suspensions etc.; and parenteral preparations such as injections (e.g., subcutaneous injection, intravenous injection, intramuscular injection, intraperitoneal injection, etc.), external preparations (e.g., preparation for nasal administration, percutaneous preparation, ointment, etc.), suppository (e.g., rectal suppository, vaginal suppository, etc.), pellets, drops, sustained-release preparations, each of which can be safely administered orally or parenterally.

The agent of the present invention can be produced by conventional methods in formulation technical fields, such as methods described in Japanese Pharmacopoeia, and the like. Specific methods for preparing formulations are described below in detail.

The oral preparation can be prepared by adding, to the active ingredients, for example, an excipient (e.g., lactose, saccharose, starch, D-mannitol, etc.), a disintegrant (e.g., carboxymethylcellulose calcium, etc.), a binder (e.g., pregelatinized starch, gum arabic, carboxymethylcellulose, hydroxypropylcellulose, polyvinylpyrrolidone, etc.) or a lubricant (e.g., talc, magnesium stearate, polyethylene glycol 6000, etc.) and compression-molding the mixture, and, if desired, subsequently coating the preparation with coating base by a method known per se, for the purpose of taste masking, enteric dissolution or sustained release.

Examples of such coating base include sugar coating base, water-soluble film coating base, enteric film coating base, sustained-release film coating base, and the like.

As the sugar coating base, saccharose is used, and additionally, one, two or more substances selected from talc, precipitated calcium carbonate, gelatin, gum arable, pullulan, Carunauba wax may be used together.

Examples of the water-soluble film coating base include cellulose polymers such as hydroxypropyl cellulose, hydroxypropyl methyl cellulose, hydroxyethyl cellulose, methyl hydroxyethyl cellulose; synthetic polymers such as polyvinylacetal diethylaminoacetate, aminoalkyl methacrylate copolymer E [Eudragit E (trade name), Rohm Pharma], polyvinylpyrrolidone; polysaccharides such as pullulan, and the like.

Examples of the enteric film coating base include cellulose polymers such as hydroxypropyl methyl cellulose phthalate, hydroxypropyl methyl cellulose acetate succinate, carboxy methyl ethyl cellulose, cellulose acetate phthalate; acrylate copolymers such as methacrylate copolymer L [Eudragit L (trade name), Rohm Pharma], methacrylate copolymer LD [Eudragit L-30D55 (trade name), Rohm Pharma], methacrylate copolymer S [Eudragit S (trade name), Rohm Pharma]; natural products such as shellac, and the like.

Examples of the sustained-release film coating base include cellulose polymers such as ethyl cellulose; acrylate polymers such as aminoalkyl methacrylate copolymer RS, [Eudragit RS (trade name), Rohm Pharma], ethyl acrylate/methyl methacrylate copolymer suspension [Eudragit NE (trade name), Rohm Pharma], and the like.

Two or more coating bases described above may be used by mixing them in an appropriate ratio. For coating, a shading agent such as titanium oxide, iron sesquioxide etc. may be used.

An injection can be produced by dissolving, suspending or emulsifying an active ingredient in an aqueous solvent (e.g., distilled water, physiological saline, Ringer's solution etc.), an oily solvent (e.g., vegetable oil such as olive oil, sesame oil, cottonseed oil, corn oil etc.; propylene glycol, etc.) and the like, together with a dispersant (e.g., polysorbate 80, polyoxyethylene-hydrogenated castor oil 60 etc.), polyethylene glycol, carboxymethyl cellulose, sodium alginate etc.), a preservative (e.g., methyl paraben, propyl paraben, benzyl alcohol, chlorobutanol, phenol etc.), an isotonizing agent (e.g., sodium chloride, glycerine, D-mannitol, D-sorbitol, glucose, etc.) and the like. Where desired, additives may be used, such as a solubilizing agent (e.g., sodium salicylate, sodium acetate etc.), a stabilizer (e.g., human serum albumin etc.), a soothing agent (e.g., benzyl alcohol etc.), and the like.

Of the various dosage forms described above, oral preparations such as tablet and capsule are particularly preferred in view of convenience in taking the medicine.

The pharmaceutical composition of the present invention is useful for decreasing disorders in tissues such as the gastrointestinal tract and the kidneys, which are observed in patients suffering from various diseases (e. g. rheumatoid arthritis, osteoarthritis, etc.) who are on medication of a compound having an activity capable of causing a tissue disorder, such as a compound having COX inhibiting activity (e.g., analgesic/anti-inflammatory agent etc.).

The pharmaceutical composition of the present invention can decrease disorders in the organs such as the gastrointestinal tract and the kidneys, which are caused by a compound having COX inhibiting activity, and the like, and observed in patients on medication of a compound having an activity capable of causing a tissue disorder (e.g. rheumatoid arthritis, osteoarthritis, etc.), such as a compound having COX inhibiting activity, by about 60% or more, preferably about 70% or more, more preferably about 80% or more, most preferably about 90% or more.

The pharmaceutical composition of the present invention, particularly, a composition comprising a compound having HSP inducing activity and COX inhibiting activity can be applied for the prophylaxis and/or treatment of diseases illustrated below:
arthritis (e.g., rheumatoid arthritis, osteoarthritis, rheumatoid myelitis, urarthritis, periostitis), asthma, allergic disease, arterial sclerosis, digestive system disease such as inflammatory enteropathy (e.g., Crohn's disease, ulcer (particularly ulcerative colitis)), diabetic complications (diabetic neuropathy and diabetic angiopathy), atopic dermatitis, chronic obstructive pulmonary diseases, systemic lupus erythematodes, organ inflammatory disease (nephritis and hepatitis), autoimmune hemolytic anemia, psoriasis, neurodegenerative diseases (e.g., Alzheimer's disease, Parkinson's disease, amyotrophic lateral sclerosis, AIDS encephalopathy), central nerve disorders (e.g., cerebrovascular diseases such as cerebral hemorrhage and brain infarction, cephalic trauma, spine damage, brain edema, multiple sclerosis), meningitis, angina pectoris; cardiac infarction, congestive heart failure, inflammatory ophthalmic diseases, inflammatory pulmonary diseases (e.g., chronic pneumonia; silicosis, pulmonary sarcoidosis, pulmonary tuberculosis), endometriosis, toxinemia (e.g., sepsis, septic shock, endotoxic shock, Gram-negative sepsis, toxic shock syndrome), cachexia (e.g., cachexia caused by infection, cancerous cachexia, cachexia caused by acquired immunodeficiency syndrome), cancer, cancer pain, Addison's disease, Creutzfeldt-Jakob disease, viral infections (e.g., infections with virus such as cytomegalo virus, influenza virus, herpes virus etc.), transplantation, disseminated intravascular coagulation syndrome, acute pain caused by inflammation, pain associated with chronic inflammation, pain after surgery (incisional pain, deep pain, visceralgia, chronic pain after surgery etc.), myalgia (myalgia associated with chronic pain disease, shoulder stiffness, etc.), arthralgia, toothache, jaw joint pain, cephalalgia (migraine, tension-type headache, cephalalgia associated with fever, cephalalgia associated with hypertension), visceralgia (cardialgia, anginal pain, abdominal pain, nephralgia, ureteralgia, cystalgia, obstetric gynecologic pain (mittelschmerz, dysmenorrhea, labor pains)), neuralgia (hernia of intervertebral disk, radiculalgia, postherpetic neuralgia, trigeminal neuralgia), reflex sympathetic dystrophy, complex local pain syndrome and the like.

Furthermore, the pharmaceutical composition of the present invention can be used in combination with agents such as agents for treating diabetes, agents for treating diabetic complications, anti-hyperlipidemia agents, antihypertensive agents, diuretics, chemotherapeutics or immunotherapeutics (hereinafter, abbreviated as combination drug). The agent of the present invention itself can comprise these combination drugs. In the present specification, unless particularly specified, a simple expression of "combination" may be administration of separate pharmaceutical agents or administration of a mixture as a single pharmaceutical agent. When separated pharmaceutical agents are used in combination, administration time period of the agent of the present invention and the combination drug is not limited, and may be administered to the administration subject simultaneously or at time interval. Additionally, two or more combination drugs may be used in combination at an appropriate ratio.

The dosage of the combination drug can be appropriately determined based on the dose clinically used for each agent. In addition, compounding ratio of the agent of the present invention and the combination drug can be determined depending on administration subject, administration route, target disease, symptom or combination thereof, and the like.

### EXAMPLES

Hereinafter; the present invention is described in more detail by reference to Production Examples, Experimental Examples, Test Examples and Examples, which are only examples, and are not intended to limit the present invention, and may be modified within the scope of the present invention.

The term "room temperature" in the following Production Examples refers to a temperature of about 10 to 35°C. The "%" refers to percent by weight unless it is explicitly indicated otherwise. Silica gel used is Kieselgel 60, 0.063-0.200 mm (Merck) unless it is explicitly indicated otherwise, and basic silica gel is Chromatorex NH-DM1020, 0.100-0.200 mm, (Fuji Silysia Chemical).

The meanings of other abbreviations used in the description are as follows
s : singlet
d : doublet
t : triplet
q : quartet
m : multiplet
br : broad
J : coupling constant
Hz : Hertz
CDCl₃ : deuterated chloroform
DMSO-d₆ : deuterated dimethylsulfoxide
NMR : Proton Nuclear Magnetic Resonance

### Production Example 1

### 2-Hydrazinopyridine

According to the method described in J. Med. Chem., 28, 1394 (1985), the title compound was prepared. A mixture of 2-chloropyridine (200 mL, 2.1 mol) and hydrazine monohydrate (400 mL, 8.2 mol) was heated and refluxed for 20 hrs. The solution was cooled to room temperature, excess hydrazine hydrate was evaporated under reduced pressure, and the residue was poured into water. The solution was made basic by the addition of a sodium hydroxide solution, and organic matter was extracted with chloroform. The extract was washed with saturated brine and water, and dried over anhydrous magnesium sulfate, and the solvent was evaporated under reduced pressure to give the title compound (157 g, 68% yield). The compound was used in the following process without further purification.

### Production Example 2

### 3-Methyl-1-(2-pyridinyl)-1H-pyrazol-5-ylamine

To an ice-cold solution of aminocrotononitrile (82 g, 1.0 mol) and 2-hydrazinopyridine (120 g, 1.1 mol) in ethanol (300 mL) was added acetic acid (132 g, 2.2 mol), and the solution, was heated at reflux for 3.5 hrs. The solution was cooled to room temperature, and the solution was concentrated under reduced pressure, and water was added to the residue. The solution was made basic by the addition of an aqueous sodium hydroxide solution, and organic matter was extracted with ethyl acetate. The extract was washed with saturated brine and water, and dried over anhydrous magnesium sulfate, and the solvent was evaporated under reduced pressure. The residue was purified by silica gel column chromatography (ethyl acetate) to give the title compound (156.3 g, 90% yield).
mp: 103-104°C (recrystallized from ethyl acetate).
NMR (CDCl₃) δ: 2.25 (3H, s), 5.37 (1H, s), 5.92 (2H, br *s),* 7.07 (1H, m), 7.76 (1H, m), 7.94 (1H, d, J=7.0Hz), 8.32 (1H, d, J=6.0Hz).

### Production Example 3

### 2-Chloro-5-(trifluoromethyl)benzoic acid

The title compound was prepared according to the method described in Tetrahedron Lett., 37, 2767 (1996). To a solution of 1-chloro-4-(trifluoromethyl)benzene (25.8 g, 143 mmol) and tetramethylene diamine (16.6 g, 143 mmol) in tetrahydrofuran (250. mL) cooled to -78°C, was added a solution of 1.6 M butyllithium in hexane (89.4 mL, 143 mmol) was added dropwise under an argon atmosphere, and the mixture was stirred at the same temperature for 30 min. The solution was carefully poured onto crushed dry ice, and the resulting mixture was allowed to warm to room temperature. The solution was concentrated under reduced pressure, and the residue was poured into water. The solution was washed with diethyl ether, and subsequently made acidic by the addition of conc. hydrochloric acid, and organic matter was extracted with dichloromethane. The extract was washed with saturated brine and dried over anhydrous magnesium sulfate, and the solvent was evaporated under reduced pressure. The residue thus obtained was crystallized from hexane to give the title compound (20.6 g, 64% yield). NMR (CDCl₃) δ: 7.65 (1H, d, J=8.4Hz), 7.75 (1H, dd, J=2.2Hz. 8.4Hz), 8.31 (1H, d, J=2.2Hz) , hidden (1H).

### Production Example 4

### 2-[[3-Methyl-1-(2-pyridinyl)-1H-pyrazol-5-yl]amino]-5-(trifluoromethyl)benzoic acid

A solution of 3-methyl-1-(2-pyridinyl)-1H-pyrazol-5-ylamine (8.71 g, 50.0 mmol), 2-chloro-5-(trifluoromethyl)benzoic acid (12.4 g, 55.0 mmol), copper acetate (II) (1.00 g, 5.50 mmol) and potassium carbonate (7.60 g, 55.0 mmol) in N,N-dimethylformamide (50 mL) was heated at reflux for 1.5 hrs. under an argon atmosphere. The solution was cooled to room temperature, and poured into water. The solution was made acidic by the addition of an acetic acid solution, and the resulting crude crystals were collected by filtration. The crystals were washed with water and air dried to give the title compound (17.7 g, 89% yield).
mp: 228-229°C (recrystallized from ethyl acetate).
NMR (CDCl₃) δ: 2.37 (3H, s), 6.19 (1H, s), 7.13 (1H, ddd,
J=1.0Hz, 4.8Hz, 7.4Hz), 7.70-7.85 (3H, m) , 7.93 (1H, d, J=8.4Hz), 8.39 (1H, d, J=1.8Hz), 8.45 (1H, ddd, J=0.8Hz, 1.8Hz, 4.8Hz), 12.46 (1H, br s) , hidden (1H).

| Elemental Analysis for C₁₇H₁₃F₃N₄O₂ | | | |
|---|---|---|---|
| Calcd. | C, 56.36; | H, 3.62; | N, 15.46. |
| Found | C, 56.56; | H, 3.52; | N, 15.63. |

### Production Example 5

### 4-Chloro-3-methyl-1-(2-pyridinyl)-6-(trifluoromethyl)-1H-pyrazolo[3,4-b]quinoline

A solution of 2-[[3-methyl-1-(2-pyridinyl)-1H-pyrazol-5-yl]amino]-5-(trifluoromethyl)benzoic acid (14.0 g, 38.6 mmol) in phosphorous oxychloride (27.4 mL, 294 mmol) was heated under reflux for 1 hr. The solution was allowed to cool to room temperature, and the reaction solvent was evaporated under reduced pressure, and the residue.was poured into iced water. The solution was neutralized by the addition of a sodium hydroxide solution, and organic matter was extracted with chloroform. The extract was washed with saturated brine and water and dried over anhydrous magnesium sulfate, and the solvent was evaporated under reduced pressure. The residue was purified by silica gel column chromatography (hexane:chloroform=1:1 to chloroform) to give the title compound (7.07 g, 50% yield).
mp: 206°C (recrystallized from ethyl acetate/methanol).
NMR (CDCl₃) δ: 3.01 (3H, s) , 7.28 (1H, ddd, J=1.0Hz, 4.8Hz, 7.4Hz), 7,91-7.99 (2H, m), 8.27 (1H, d, J=9.2Hz), 8.68-8.77 (3H, m).

| Elemental Analysis for C₁₇H₁₀ClF₃N₄ | | | | | |
|---|---|---|---|---|---|
| Calcd. | C, 56.29; | H, 2.78; | N, 15.45; | Cl, 9.77; | F, 15.71. |
| Found | C, 56.23; | H, 3.00; | N, 15.23; | Cl, 9.62; | F, 15.70. |

### Production Example 6

### 3-Methyl-1-(2-pyridinyl)-6-(trifluoromethyl)-1,9-dihydro-4H-pyrazolo[3,4-b]quinolin-4-one (Compound A)

To a solution of 4-chloro-3-methyl-1-(2-pyridinyl)-6-(trifluoromethyl)-1H-pyrazolo[3,4-b]quinoline (6.50 g, 17.9 mmol) in ethanol (300 mL), 6N hydrochloric acid (10 mL, 60 mmol) was added and the mixture was heated at reflux for 5 hrs. The solution was allowed to cool to room temperature, and the resulting crystals were collected by filtration. The crystals were washed with ethanol and air dried, and recrystallized from ethanol to give the title compound (4.69 g, 76% yield).
mp: 250-251°C (recrystallized from ethanol).
NMR (CDCl₃) δ: 2.74 (3H, s), 7.26 (1H, ddd, J=1.2Hz, 5.0Hz, 7.2Hz), 7.53 (1H, d, J=8.8Hz), 7.84 (1H, dd, J-2.0Hz, 8.8Hz), 7.92 (1H, ddd, J=1.8Hz, 7.2Hz, 8.4Hz), 8.03 (1H, ddd, J=1.0Hz, 1.2Hz, 8.4Hz), 8.49 (1H, ddd, J=1.0Hz, 1.8Hz, 5.0Hz) , 8.75 (1H, d, J=2.0Hz), 11.65 (1H, br s).

| Elemental Analysis for C₁₇H₁₁F₃N₄O | | | | |
|---|---|---|---|---|
| Calcd. | C, 59.31; | H, 3.22; | N, 16.27; | F, 16.55. |
| Found | C, 59.23; | H, 3.40; | N, 16.00; | F, 16.59. |

### Production Example 7

### 4,5-Difluoro-2-[[3-methyl-1-(2-pyridinyl)-1H-pyrazol-5-yl]amino]benzoic acid

Under an argon atmosphere, a solution of 3-methyl-1-(2-pyridinyl)-1H-pyrazol-5-ylamine (6.01 g, 34.5 mmol), 2-chloro-4,5-difluorobenzoic acid (6.64 g, 34.5 mmol), copper acetate (II) (0.718 g, 3.95 mmol) and potassium carbonate (4.77 g, 34.5 mmol) in N,N-dimethylformamide (30 mL) was heated under reflux for 2 hrs. The solution was cooled to room temperature, and poured into water. The solution was made weakly acidic by the addition of an acetic acid solution, and the resulting crude crystals were collected by filtration. The crystals were washed with water and air dried to give the title compound (9.21 g, 81% yield).
mp: 247-248°C (recrystallized from methanol).
NMR (DMSO-d₆) δ: 2.25 (3H, s), 6.33 (1H, s), 7.28-7.34 (1H, m), 7.46-7.56 (1H, m) , 7.80-8.02 (3H, m) , 8.30-8.44 (1H, m), 12.30 (1H, br s), hidden (1H).

| Elemental Analysis for C₁₆H₁₂F₂N₄O₂ | | | |
|---|---|---|---|
| Calcd. | C, 58.18; | H, 3.66; | N, 16.96. |
| Found | C, 5.8:09; | H, 3.48; | N, 16.88. |

### Production Example 8

### 4-Chloro-6,7-difluoro-3-methyl-1-(2-pyridinyl)-1H-pyrazolo[3,4-b]quinoline

A solution of 4,5-difluoro-2-[[3-methyl-1-(2-pyridinyl)-1H-pyrazol-5-yl]amino]benzoic acid (8.14 g, 24.6 mmol) in phosphorous oxychloride (13.mL, 138 mmol) was heated under reflux for 1.5 hrs. The.solution was cooled to room temperature and concentrated under reduced pressure, and the residue was poured into iced water. The solution was neutralized by the addition of a sodium hydroxide solution,. and organic matter was extracted with chloroform. The extract was washed with saturated brine and water, and dried over anhydrous magnesium sulfate, and the solvent was evaporated under reduced pressure. The residue thus obtained was purified by silica gel column chromatography (chloroform) to give the title compound (4.42 g, 54% yield).
mp: 175-176°C (recrystallized from ethyl acetate).
NMR (CDCl₃) δ: 2.98 (3H, s), 7.23-7.30 (1H, m) , 7.87-7.98 (2H, m), 8.13 (1H, dd,.J=8.6Hz, 10.8Hz), 8.67-8.72 (2H, m).

| Elemental Analysis for C₁₆H₉ClF₂N₄ | | | | | |
|---|---|---|---|---|---|
| Calcd. | C, 58.11; | H, 2.74; | N, 16.94;. | Cl, 10.72; | F, 11.49. |
| Found | C, 57.71; | H, 2.77; | N, 16.90; | Cl, 10.50; | F, 11.17. |

### Production Example 9

### 6,7-Difluoro-3-methyl-1-(2-pyridinyl)-1,9-dihydro-4H-pyrazolo[3,4-b]quinolin-4-one (Compound B)

To a solution of 4-chloro-6,7-difluoro-3-methyl-1-(2-pyridinyl)-1H-pyrazolo[3,4-b]quinoline (2.21 g, 6.68 mmol) in ethanol (120 mL), 6N hydrochloric acid (6 mL, 36.0 mmol) was added and the mixture was heated under reflux for 3 hrs. The solution was allowed to cool to room temperature, and the resulting crystals were collected by filtration. The crystals were washed with ethanol and air dried to give the title compound (2. 08g, 99% yield).
mp: 274-277°C (recrystallized from ethanol).
NMR (DMSO-d₆) δ: 2.57 (3H, s) , 7.40 (1H, ddd, J=1.0Hz, 5.2Hz, 7.4Hz), 7.84-8.11 (3H, m), 8.17 (1H, dd, J=7.0Hz, 12.4Hz) , 8.57-8.60 (1H, m) , 12.04 (1H, s) .

| Elemental Analysis for C₁₆H₁₀F₂N₄O | | | | |
|---|---|---|---|---|
| Calcd. | C, 61.54; | H, 3.23; | N, 17.94; | F, 12.17. |
| Found | C, 61.45; | H, 3.00; | N, 17.77; | F, 12.20. |

### Production Example 10

### 5-Chloro-4-fluoro-2-[[3-methyl-1-(2-pyridinyl)-1H-pyrazol-5-yl]amino]benzoic acid.

Following the procedure described in Production Example 7, the title compound was prepared from 2,5-dichloro,-4-fluorobenzoic acid and 3-methyl-1-(2-pyridinyl)-1H-pyrazol-5-5 ylamine (95% yield).
mp: 250-252°C (recrystallized from ethanol).
NMR (DMSO-d₆) δ: 2.26 (3H, s), 6.38 (1H, s), 7.32 (1H, ddd, J=1.2Hz, 4.8Hz, 7.4Hz), 7.49 (1H, d, J=12.4Hz), 7.83 (1H, ddd, J=0.8Hz, 1.2Hz, 8.4Hz), 7.99 (1H, ddd, J=1.8Hz, 7.4Hz, 8.4Hz), 8.03 (1H, d, J=8.8Hz), 8.42 (1H; ddd, J=0.8Hz, 1.8Hz, 4.8Hz), 12.41 (1H, br s) , hidden (1H).

| Elemental Analysis for C₁₆H₁₂ClFN₄O₂ | | | |
|---|---|---|---|
| Calcd. | C, 55.42; | H, 3.49; | N, 16.16. |
| Found | C, 55.37; | H, 3.44; | N, 16.20. |

### Production Example 11

### 4,6-Dichloro-7-fluoro-3-methyl-1-(2-pyridinyl)-1H-pyrazolo[3,4-b]quinoline

Following the procedure described in Production Example 8, the title compound was prepared from 5-chloro-4-fluoro-2-[[3-methyl-1-(2-pyridinyl)-1H-pyrazol-5-yl]amino]benzoic acid (58% yield).
mp: 211°C (recrystallized from ethyl acetate).
NMR (CDCl₃) δ: 2.98 (3H, s), 7.23-7.30 (1H, m), 7.88 (1H, d, J=10.2Hz), 7.94 (1H, ddd, J=1. 8Hz, 7.4Hz, 8.4Hz), 8.46 (1H, d, J=7.6Mz), 8.67-8.72 (2H, m).

| Elemental Analysis for C₁₆H₉Cl₂FN₄ | | | | | |
|---|---|---|---|---|---|
| Calcd. | C, 55.35; | H, 2.61; | N, 16.14; | ;Cl, 20.42; | . F, 5.47. |
| Found | C, 55.33; | H, 2.35; | N, 16.15; | ; Cl, 20.31; | F, 5.35. |

### Production Example 12

### 6-Chloro-7-fluoro-3-methyl-1-(2-pyridinyl)-1,9-dihydro-4H-pyrazolo[3,4-b]quinolin-4-one (Compound C)

Following the procedure described in Production Example 9, the title compound was prepared from 4,6-dichloro-7-fluoro-3=methyl-1-(2-pyridinyl)-1H-pyrazolo[3,4-b]quinoline (79% yield).
mp: 284°C (recrystallized from ethanol).
NMR (DMSO-d₆) δ: 2.54 (3H, s), 7.40 (1H, ddd, J=1.0Hz, 4.8Hz, 7.4Hz), 7.84 (1H, d, J=8.4Hz), 8.05 (1H, ddd, J=1.8Hz, 7.4Hz, 8.4Hz), 8.09 (1H, d, J=9.0Hz), 8.14 (1H, d, 6.6Hz), 8.56 (1H, ddd, J=0.8Hz, 1.8Hz, 4.8Hz), 12.02 (1H, br s).

| Elemental Analysis for C₁₆H₁₀ClFN₄O | | | | | |
|---|---|---|---|---|---|
| Calcd. | C, 58.46; | H, 3.07; | N, 17.04; | Cl, 10.78; | F, 5.78. |
| Found | C, 58.38; | H, 2.97; | N, 17.14; | Cl, 10.76; | F, 5.76. |

### Production Example 13

### Ethyl 5-amino-1-(2-pyridinyl)-1H-pyrazole-4-carboxylate

A solution of ethyl 2-(ethoxymethylene)-2-cyanoacetate (33.8 g, 200 mmol) and 2-hydrazinopyridine (21.8 g, 200 mmol) in ethanol (100 mL) was heated under reflux for 20 min. The solution was cooled to room temperature, and the resulting crystals were collected by filtration. The crystals were washed with ethanol and air dried, yielding the title compound (33.8 g, 73% yield).
mp: 103-104°C (recrystallized from ethyl acetate).
NMR (CDCl₃) δ: 1. 37 (3H, t, J=7.0Hz), 4.31 (2H, q, J=7.0Hz), 7.12-7.18 (1H, m), 7.48 (2H, br s), 7.76 (1H, s), 7.77-7.87 (1H, m), 7.95 (1H, d, J= 8.4Hz), 8.35-8.38 (1H, m).

### Production Example 14

### 1-(2-Pyridinyl)-1H-pyrazol-5-ylamine

A suspension of ethyl 5-amino-1-(2-pyridinyl)-1H-pyrazole-4-carboxylate (27.9 g, 120 mmol) in 4N sodium hydroxide (300 mL) solution was heated under reflux for 1 hr. The mixture was cooled to room temperature, neutralized with conc. hydrochloric acid, and made acidic with acetic acid. The resulting crystals were collected by filtration, washed with ethanol, and air dried. The crystals were subjected to heat at 200°C, and then washed with diethyl ether, yielding the title compound (6.02 g, 31% yield).
NMR (CDCl₃) δ: 5.51 (1H, d, J=1.8Hz), 5.95 (2H, br s), 7. 07-7.13 (1H, m), 7.42 (1H, d,J=1.8Hz), 7. 75-7. 84 - (1H, m) ; 7. 98 (1H, d, J=8.4Hz), 8.33 (1H, dd, J=1.6Hz, 4.6Hz).

### Production example 15

### 5-Chloro-2-[[1-(2-pyridinyl)-1H-pyrazol-5-yl]amino]benzoic acid

Following the procedure described in Production Example 7, the title compound was prepared from 5-chloro-2-iodobenzoic acid and 1-(2-pyridinyl)-1H-pyrazol-5-ylamine (88% yield).
mp: 233-234°C (recrystallized from ethanol).
NMR (DMSO-d₆) δ: 6.42 (1H, d, J=2.0Hz), 7.38 (1H, ddd, J=1.2Hz, 4.8Hz, 7.4Hz), 7.54 (1H, dd, J=2.6Hz, 8.8Hz), 7.63 (1H, d, J=8.8Hz), 7.71 (1H, d, J=2.0Hz), 7.88-7.92 (2H, m), 7.99-8.08 (1H, m), 8.47 (1H, dd, J=0.8Hz, 1.8Hz, 4.8Hz), 12.24 (1H, br s), hidden (1H).

| Elemental Analysis for C₁₅H₁₁ClN₄O₂ | | | |
|---|---|---|---|
| Calcd. | C, 57.24; | H, 3.52; | N, 17.80. |
| Found | C, 57.13; | H, 3.46; | N, 17.72. |

### Production Example 16

### 4,6-Dichloro-1-(2-pyridinyl)-1H-pyrazolo[3,4-b]quinoline

Following the procedure described in Production Example 8, the title compound was prepared from 5-chloro-2 -[[1-(2-pyridinyl)-1H-pyrazol-5-yl]amino]benzoic acid (25% yield):
mp: 175-176°C (recrystallized from ethyl acetate).
NMR (CDCl₃) δ: 7.28-7.34 (1H, m), 7.77 (1H, dd, J=2.4Hz, 9.0Hz), 7.93-8.01 (1H, m), 8.18 (1H, d, J=9.0Hz), 8.37 (1H, d, J=2.4Hz), 8. 59 (1H, s) , 8.66-8.71 (2H, m) .

| Elemental Analysis for C₁₅H₈Cl₂N₄.0.5H₂O | | | | |
|---|---|---|---|---|
| Calcd. | C, 55.58; | H, 2.80; | N, 17.28; | Cl, 21.87. |
| Found | C, 55.42; | H, 2.74; | N, 17.25; | Cl, 21.80. |

### Production Example 17

### 6-Chloro-1-(2-pyridinyl)-1,9-dihydro-4H-pyrazolo[3,4-b]quinolin-4-one (Compound D)

Following the procedure described in Production Example 2-13, the title compound was prepared from 4,6-dichloro-1-(2-pyridinyl)-1H-pyrazolo[3,4-b]quinoline (76% yield).
mp: 297-298°C (recrystallized from ethanol).
NMR (DMSO-d₆) δ: 7.46 (1Hm, ddd, J=1.0Hz, 5.0Hz, 7.4Hz), 7.79 (1H, dd, J=2.6Hz, 8.8Hz), 7.97 (1H, d, J=8.0Hz), 8.07-8.20 (3H, m), 8.40 (1H, s), 8.63-8.66 (1H, m), 11.89 (1H, br s).

| Elemental Analysis for C₁₅H₉ClN₄O | | | | |
|---|---|---|---|---|
| Calcd. | C, 60.72; | H, 3.06; | N, 18.88; | Cl, 11.95. |
| Found | C, 60.52; | H, 3.04; | N, 18.79; | Cl, 11.89. |

### Experimental Example

The gene manipulation methods described in Experimental Example below are carried out in accordance with the methods described in Maniatis et al., Molecular Cloning (Cold Spring Harbor Laboratory, 1989) or the methods described in the protocols attached to reagents.

### Preparation of microsome fractions

### Preparation of human COX-1 cDNA recombinant baculovirus

A 1.8 kb DNA fragment containing human COX-1 cDNA (FASEB J., 5.(9), 2304-2312 (1991)) prepared by the PCR method was inserted into plasmid pFASTBAC1 (CIBCOBRL) to obtain a plasmid pFBCOX1.

Using the plasmid pFBCOX1 and BAC-TO-BAC Baculovirus Expression System (GIBCOBRL), a virus stock BAC-COX1 of the recombinant baculovirus was prepared. Preparation of microsome fractions from COX-1 expressing insect cells

Sf-21 cells were inoculated in 125 mL of Sf-900 II SFM medium (GIBCOBRL) at a concentration of 1×10⁶ cells/mL, and the medium was incubated at 27°C for 24 hrs. 0.75 mL of the virus stock BAC-COX1 of the recombinant baculovirus was added, and the mixture was incubated for additional 72 hrs. The cells were separated from the medium by centrifugation (3000. rpm, 10 min.)., and washed with PBS twice. The cells were suspended in 10 mL of a lysis buffer (0.1 M Tris-HCl (pH 7.4), 5 mM EDTA) , and were treated with a homogenizer (POLYTRON) three times at 20000 rpm for 20 seconds so that the cells were crushed. The supernatant obtained after centrifugation (2000 rpm, 10 min.) was further centrifuged (40000 rpm, 45 min.) to give a uprecipitate, which was resuspended in a Lysis buffer (0.1 M Tris-HCl (pH 7.4), 5 mM EDTA), and the suspension was stored at -80°C.

### Preparation of human COX-2 cDNA recombinant baculovirus

A 1.8 kb DNA fragment containing human COX-2 cDNA (Proc. Natl. Acad. Sci. U.S.A., 89 (16), 7384-7388 (1992)) prepared by the PCR method was inserted into plasmid pFASTBAC1 (CIBCOBRAL) to obtain a plasmid pFBCOX2.

Using the plasmid pFBCOX2 and BAC-TO-BAC Baculovirus Expression System (GIBCOBRAL), a virus stock BAC-COX2 of the recombinant baculovirus was prepared.

### Preparation of microsome fractions from COX-2 expressing insect cells

Sf-21 cells were inoculated in 125 mL of LSf-900 II SFM medium (GIBCOBRAL) at a concentration of 1×10⁶ cells/mL, and the medium was incubated at 27°C for 24 hrs. 0.75 mL of the virus stock BAC-COX2 of the recombinant baculovirus was added, and was incubated for additional 72 hrs. The cells were separated from the medium by centrifugation (3000 rpm, 10 min.), and washed twice with PBS. The cells were suspended in 10 mL of a Lysis buffer (0.1 M Tris-HCl (pH 7.4), 5 mM EDTA) , and were treated with a homogenizer (POLYTRON) , three times at 20000 rpm for 20 sec. so that the cells were crushed. The supernatant thus obtained after centrifugation (2000 rpm, 10 min.) was further centrifuged (40000 rpm, 45 min.) to obtain a precipitate, which was resuspended in a Lysis buffer (0.1 M Tris-HCl (pH 7.4), 5 mM EDTA), and the suspension was stored at - 80°C.

### Test Example 1

### COX inhibiting activities

5 To a mixture of 20 mL of a reaction buffer previously concentrated 10 times (1 M Tris-HCl (pH 8.0), 50 mM EDTA, 1.0% Tween 20, 50, mM luminol, 100 mM hematin) , 20 mL of the microsome fractions (COX-1: 40.mg, COX-2: 20 mg), and 55 mL of distilled water, a sample compound dissolved in DMF (5 mL) was added, and the mixture was left at 37°C for 25 min. The reaction was started by the addition of 20 mM arachidonic-acid (100 mL), and chemiluminescence amount during the 10-second period after the addition of arachidonic acid was determined by the use of Lumistar (BMG Lab technologies, GmbH). The inhibition rate was calculated, regarding that the enzyme activity when DMF (5 mL) was added was 100%, and the enzyme activity was 0% when 4 mM flurbiprofen (5 mL) was added. The results are shown in Table 1.

**Table 1**

| Compound | COX-1 inhibiting activity (IC₅₀) | COX-2 inhibiting. activity (IC₅₀) |
|---|---|---|
| Compound A | 0.45 µM | 0.38 µM |
| Compound B | 0.527 µM | 0.295 µM |
| Compound C | 0.263 µM | 0.213 µM |
| Compound D | 0.600 µM | 0.360 µM |

The results of table 1 shows that Compounds A, B, C and D have excellent COX inhibiting activities.

### Test Example 2

### Acetic acid rising method

Male ICR-mice (5 weeks old, CLEA Japan) were used (1 group, 10 mice), and a sample was orally administered (0.2 mL/10 g, b.w.). After 30 min., 0.6% acetic acid solution was injected to mice intraperitoneally (0.1 mL/10 g, b.w.), and the mice were immediately transferred into an observation cage made of a transparent acrylic resin. The number of mice rising and stretching during the following period of 20 min. was counted. The repression rate (%) was calculated by comparing the average numbers between the sample groups and the control groups and ID₅₀ value was calculated. The results are shown in Table 2.

**Table 2**

| | |
|---|---|
| Compound | ID₅₀ |
| Compound A Compound A | 0.9 mg/kg |

The result in Table 2 shows that Compound A has an excellent analgesic activity.

### Test Example 3

### carrageenin edema test

Male SD rats (6-week-old, CLEA Japan) were used (6 rats per group). The carrageenin edema test was performed in accordance with the method of Winter et al. (Proc. Soc. Exp. Biol. Med.; 111, 544-547, 1962). The volume of the footpad of the right hind limb was measured. Then, the test compound was orally administered (1.0 mL/100 g body weight), and immediately thereafter, water was orally administered to 5 mL/rat. Vehicle alone was orally administered to control group. After 1 hr., 0.05 mL of saline containing 1% carrageenin was subcutaneously injected into the footpad of the right hind limb to induce edema. After 2 hr. and 3 hr., the volume of the footpad of the right hind limb was measured. For the effects of the compound, the difference in the volume of the foot before and 2 hr. and 3 hr. after the carrageenin injection was determined, from which inhibitory rate (%) relative to the control group was calculated, and ID₃₀ was determined. The result is shown in Table 3.

**Table 3**

| | |
|---|---|
| Compound | ID₃₀ |
| Compound A | 2.2 mg/kg |

The result of Table 3 shows that Compound A has an excellent anti-inflammatory activity.

### Test Example 4

### HSP27-inducing activity

Compound A (30 mg/ml) and vehicle were orally administered to rats. After 2 hr., stomach was excised from the rats, opened and washed with ice-cooled physiological saline. Gastric mucosa was taken from the stomach using sterilized cover glass, and immediately suspended in ISOGEN (Wako Pure Chemical Industries, Ltd.). Total RNA was prepared from the gastric mucosa according to instructions, and purified by RNAeasy Midi Kit (QIAGEN). Then cDNA was synthesized from the total RNAusing TaqMan Gold RT-PCR Kit (Applied Biosystems), and subjected to TaqMan PCR using ABI7700 (Applied Biosystems), based on which the amount of HSP 27 gene expression was determined. Forward primer used was 5'-taagaccaaggaaggcgtggt-3' (SEQ ID NO:1)., Reverse primer used was 5'-ccgagagatgtagccatgttca-3' (SEQ ID NO:2), and TaqMan probe used was 5'-tcactggcaagcacgaagaaaggcag-3' (SEQ ID NO:3) labeled with FAM (6-carboxyfluorescein). Additionally, the amount of GAPDH gene expression was determined using TaqMan Rodent GAPDH Control Reagents VIC probe (Applied Biosystems) and G3PDH cDNA Control probe (CLONTECH), and the amount was employed as an internal control. The amount of HSP27 gene expression is shown by the values calibrated with an amount of internal control (i.e., GAPDH gene) expression (Table-4). HSP27 gene expression of the Compound A administration group showed an increase as compared to the vehicle administration group.

**Table 4**

| Experimental Group | HSP27 / GAPDH (x 10⁴) (% of control) |
|---|---|
| Vehicle | 71 ± 13 (100) |
| Compound A | 197 ± 20 (277) |

### Test Example 5

### Test for gastric mucosa disorder

Male SD rats (6 rats, 7 weeks old, CLEA Japan) were used. The test compound was orally administered (5 mL/kg, b.w.) to the rats after 24 hr-fasting or without fasting. After 23.5 hrs., the rats were intravenously injected with Evan's Blue (0.5% Evan's Blue/saline, 1 mL/rat) without anesthesia. After. 30, min., the rats were laparotomized and stomach was excised. Esophagus was clipped. A 1% formalin solution (8 mL) was infused to the esophagus, and duodenum was clipped. The stomach was immersed in a 1% formalin solution for 10 min. or more, and sectioned along the line from duodenum to greater curvature of the stomach. After washing the stomach, the total length (mm²) of the site (hemorrhage site: detachment of mucosa - ulcer) stained with Evan's Blue was measured with a counter under a stereomicroscope and recorded. The results are shown in Table 5.

**Table 5**

| | Area of lesion (mm²) (average) | |
|---|---|---|
| Experimental group | Administration without fasting | Administration after 24 hr-fasting |
| Compound A (300 mg/kg). | 0 | 0.2 |

### Example 1

| | |
|---|---|
| (1) Compound A | 10.0 g |
| (2) Lactose | 60.0 g |
| (3) Cornstarch | 35.0 g |
| (4) Gelatin | 3.0 g |
| (5) Magnesium stearate | 2.0 g |

A mixture of Compound A (10. 0 g), lactose (60.0 g), and cornstarch (35.0 g) was sieved through a 1 mm mesh sieve by the use of an aqueous solution of 10 wt% gelatin (30 ml, 3.0.g as gelatin), and the resulting granules were dried at 40°C, and sieved once again. The granules thus obtained were mixed with magnesium stearate (2.0 g) and compressed. The core tablet thus obtained was sugarcoated by the use of an aqueous suspension containing sucrose, titanium dioxide, talc and gum acacia. The coated tablets were glazed with beeswax to give 1000 coated tablets.

### Example 2

| | |
|---|---|
| (1) Compound A | 10.0 g |
| (2) Lactose | 70.0 g |
| (3) Cornstarch | 50.0 g |
| (4) Soluble starch | 7.0 g |
| (5) Magnesium stearate | 3.0 g |

A mixture of Compound A (10.0 g) and magnesium stearate (3.0 g) was granulated by the use of an aqueous solution of soluble starch (70 ml, 7.0 g as soluble starch), and the resulting granules were dried, and mixed with lactose (70.0 g) and cornstarch (50.0 g). The mixture was compressed'to give 1000 tablets:

### Industrial Applicability

In the treatment of rheumatoid arthritis, osteoarthritis and other diseases, administration of a compound having COX inhibiting activity, which is effective for the treatment, tends to advance healing of the target disease but cause disorders of gastrointestinal tract, kidney and the like of patients. However, the pharmaceutical composition of the present invention can decrease disorders in the organs, such as gastrointestinal tract and kidney, of such patients. In addition, a pharmaceutical agent having both HSP inducing activity and COX inhibiting activity can significantly decrease disorders in the organs, such as gastrointestinal tract and kidney, of patients, while advancing the treatment of rheumatoid arthritis or osteoarthritis. Similarly, when a "compound having HSP inducing activity" and a "compound having an activity capable of causing a tissue disorder" are to be used in combination, administration of a compound having an activity capable of causing a tissue disorder, which is effective for the treatment, in the treatment of various diseases tends to advance healing of the target disease but cause disorders of gastrointestinal tract, kidney and the like of patients. However, the pharmaceutical composition of the present invention can decrease disorders in the organs, such as gastrointestinal tract and kidney, of such patients. Furthermore, when a "compound having HSP inducing activity and an activity capable of causing a tissue disorder" is to be administered, too, it can significantly decrease disorders in the organs such as gastrointestinal tract (e.g., stomach, duodenum, small intestine etc.) and kidney of patients, while advancing the treatment of various diseases.

This application is based on a patent application No. 2001-92704 filed in Japan, the contents of which are all hereby incorporated by reference.

### Sequence Listing Free Text

SEQ ID NO:1: PCR primer for amplifying HSP27 gene
SEQ ID NO:2: PCR primer for amplifying HSP27 gene
SEQ ID NO:3: TaqMan probe

## Claims

1. An agent for inducing HSP, comprising a compound represented by the formula (I): 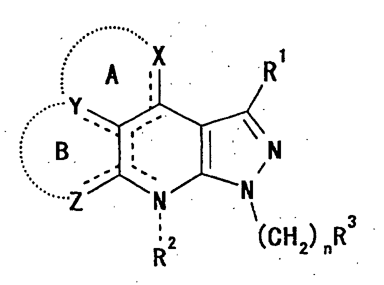 wherein
R¹ is a hydrogen atom, a hydrocarbon group which may be substituted, an amino group which may be substituted, a sulfur atom which may be substituted or a carboxyl group which may be esterified or amidated;
R² is absent, a hydrogen atom or a hydrocarbon group which may be substituted;
R³ is a heterocyclic group which may be substituted;
X, Y and Z are, respectively, a hydrogen, a halogen, a nitrile, a hydrocarbon group which may be substituted, a carboxyl group which may be esterified or amidated, an acyl group which may be substituted, -NR⁴R⁵, an oxygen atom, -OR⁴, a sulfur atom or -SR⁴ (R⁴ and R⁵ are, respectively, a hydrogen atom, a hydrocarbon group which may be substituted, or a heterocyclic group which may be substituted, or may bind to each other to form acyclic amino group together with the nitrogen atom they bind with), or X and Y may bind to each other to form ring A, or Y and Z may bind to each other to form ring B;
bond portions indicated by a solid line and a broken line are, respectively, a single bond or a double bond, and bond portions indicated by a broken line are, respectively, a single bond or absent;
ring A is a homocyclic or heterocyclic 5- to 7-membered ring which may be substituted;
ring B is a homocyclic or heterocyclic 5- to 7-membered ring which may be substituted; and
n is an integer of 0 or 1,
or a salt thereof.

2. An agent for inducing HSP, comprising 3-methyl-1-(2-pyridinyl)-6-trifluoromethyl-1,9-dihydro-4H-pyrazolo[3,4-b]quinolin-4-one or a salt thereof.

3. An agent for inducing HSP, comprising 6,7-difluoro-3-methyl-1-(2-pyridinyl)-1,9-dihydro-4H-pyrazolo[3,4-b]quinolin-4-one or a salt thereof.

4. An agent for inducing HSP, comprising 6-chloro-7-fluoro-3-methyl-1-(2-pyridinyl)-1,9-dihydro-4H-pyrazolo[3,4-b]quinolin-4-one or a salt thereof.

5. An agent for inducing HSP, comprising 6-chloro-1-(2-pyridinyl)-1,9-dihydro-4H-pyrazolo[3,4-b]quinolin-4-one or a salt thereof.

6. The agent of any of claims 1 to 5, which is an agent for ameliorating, or an agent for decreasing the progression of, a tissue disorder.

7. The agent of claim 6, wherein the tissue disorder is a digestive tract disorder or a kidney disorder.

8. A method for inducing HSP, which comprises administering an effective amount of the compound of any of claims 1 to 5, or a salt thereof to a mammal.

9. Use of the compound of any of claims 1 to 5, or a salt thereof, for the production of an agent for inducing HSP.

10. A pharmaceutical composition which decreases a tissue disorder, comprising a compound having HSP inducing activity and an activity capable of causing the tissue disorder.

11. The composition of claim 10, wherein the activity capable of causing the tissue disorder is COX inhibiting activity.

12. The composition of claim 11; which is used for inhibiting COX.

13. The composition of any of claims 10 to 12, which is an agent for ameliorating, or an agent for decreasing the progression of, the tissue disorder.

14. The composition of claim 13, wherein the tissue disorder is a gastrointestinal tract disorder or a kidney disorder.

15. An agent for ameliorating, or an agent for decreasing the progression of, a gastrointestinal tract disorder or a kidney disorder, which comprises a compound having HSP inducing activity and an activity capable of causing the gastrointestinal tract disorder or the kidney disorder.

16. The composition of claim 11 or 12, which is an agent for the prophylaxis or treatment of inflammatory disease, arthritis, rheumatism, rheumatoid arthritis or osteoarthritis.

17. An agent for the prophylaxis or treatment of inflammatory disease, arthritis, rheumatism, rheumatoid arthritis or osteoarthritis, which decreases a gastrointestinal tract or kidney disorder, said agent comprising a compound having HSP inducing activity and COX inhibiting activity.

18. The composition of claim 10, which decreases a tissue disorder caused by an activity capable of causing the tissue disorder, by about 60% or more.

19. The composition of claim 11, which decreases a tissue disorder caused by COX inhibiting activity, by about 60% or more.

20. The composition of claim 10, wherein the compound is represented by the formula (I): 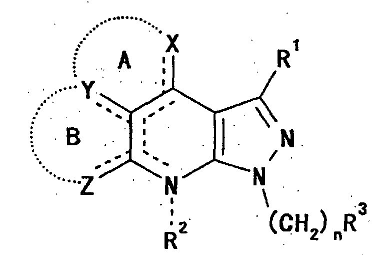 wherein
R¹ is a hydrogen atom, a hydrocarbon group which may be substituted, an amino group which may be substituted, a sulfur atom which may be substituted or a carboxyl
group which may be esterified or amidated;
R² is absent, a hydrogen atom or a hydrocarbon group which may be substituted;
R³ is a heterocyclic group which may be substituted;
X, Y and Z are, respectively, a hydrogen, a halogen, a nitrile, a hydrocarbon group which may be substituted, a carboxyl group which may be esterified or amidated, an acyl group which may be substituted, -NR⁴R⁵, an oxygen atom, -OR⁴, a sulfur atom or -SR⁴ (R⁴ and R⁵ are, respectively, a hydrogen atom, a hydrocarbon group which may be substituted, or a heterocyclic group which may be substituted, or may bind to each other to form a cyclic amino group together with the nitrogen atom they bind with), or X and Y may bind to each other to form ring A, or Y and Z may bind to each other to form ring B;
bond portions indicated by a solid line and a broken line are, respectively, a single bond or a double bond, and bond portions indicated by a broken line are, respectively, a single bond or absent;
ring A is a homocyclic or heterocyclic 5- to 7-membered ring which may be substituted;
ring B is a homocyclic or heterocyclic 5- to 7-membered ring which may be substituted; and
n is an integer of 0 or 1,
or a salt thereof.

21. The composition of claim 20, wherein the compound is 3-methyl-1-(2-pyridinyl)-6-trifluoromethyl-1,9-dihydro-4H-pyrazolo[3,4-b]quinolin-4-one or a salt thereof.

22. The composition of claim 20, wherein the compound is 6,7-difluoro-3-methyl-1-(2-pyridinyl)-1,9-dihydro-4H-pyrazolo[3,4-b]quinolin-4-one or a salt thereof.

23. The composition of claim 20, wherein the compound is 6-chloro-7-fluoro-3-methyl-1-(2-pyridinyl)-1,9-dihydro-4H-pyrazolo[3,4-b]quinolin-4-one or a salt thereof.

24. The composition of claim 20, wherein the compound is 6-chloro-1-(2-pyridinyl)-1,9-dihydro-4H-pyrazolo[3,4-b]quinolin-4-one or a salt thereof.

25. A method for decreasing a tissue disorder, which comprises administering an effective amount of a compound having HSP inducing activity and an activity capable of causing the tissue disorder to a mammal.

26. Use of a compound having HSP inducing activity and.an activity capable of causing a tissue disorder, for the production of a pharmaceutical composition which decreases the tissue disorder.

27. A method for decreasing a tissue disorder caused by COX inhibiting activity, which comprises administering an effective amount of a compound having HSP inducing activity and COX inhibiting activity to a mammal.

28. Use of a compound having HSP inducing activity and COX inhibiting activity, for the production of a pharmaceutical composition which decreases an activity capable of causing a tissue disorder caused by COX inhibiting activity.

29. A pharmaceutical composition comprising a combination of a compound having HSP inducing activity and a compound having an activity capable of causing a tissue disorder.

30. A pharmaceutical composition comprising a combination of a compound having HSP inducing activity and a compound having COX inhibiting activity.

31. The composition of claim 29 or 30, which is an agent for ameliorating, or an agent for decreasing the progression of, the tissue disorder.

32. The composition of claim 31, wherein the tissue disorder is a gastrointestinal tract or kidney disorder.

33. A method for decreasing a tissue disorder, which comprises administering an effective amount of a compound having HSP inducing activity and an effective amount of a compound having an activity capable of causing the tissue disorder to a mammal.

34. Use of a compound having HSP inducing activity and a compound having an activity capable of causing a tissue disorder, for the production of a pharmaceutical composition which decreases the tissue disorder.

35. A method for decreasing a tissue disorder caused by COX inhibiting activity, which comprises administering an effective amount of a compound having HSP inducing activity and an effective amount of a compound having COX inhibiting activity to a mammal.

36. Use of a compound having HSP inducing activity and a compound having COX inhibiting activity, for the production of a pharmaceutical composition which decreases an activity capable of causing a tissue disorder caused by COX inhibiting activity.


SEQUENCE LISTING

<110> Takeda Chemical Industries, Ltd.

<120> HSP inducing agent

<130> 09462

<150> JP 2001-92704
<151> 2001-03-28

<160> 3

<210> 1
<211> 21
<212> DNA
<213> Artificial Sequence

<220>
<223> PCR primer for amplifying HSP27 gene

<400> 1
taagaccaag gaaggcgtgg t     21

<210> 2
<211> 22
<212> DNA
<213> Artificial Sequence

<220>
<223> PCR primer for amplifying HSP27 gene

<400> 2
ccgagagatg tagccatgtt ca     22

<210> 3
<211> 26
<212> DNA
<213> Artificial Sequence

<220>
<223> TaqMan probe

<400> 3
tcactggcaa gcacgaagaa aggcag     26